# EUROPÄISCHE PATENTSCHRIFT

(11) **EP 4 327 761 B1**
(45) Veröffentlichungstag und Bekanntmachung des Hinweises auf die Patenterteilung: **15.10.2025**
(21) Anmeldenummer: 22192133.1
(22) Anmeldetag: 25.08.2022
(51) Int. Cl.: A61B 17/22, A61B 17/00

(54) **DRUCKWELLENGERÄT MIT DOPPELVENTILEINRICHTUNG**
PRESSURE WAVE DEVICE WITH DOUBLE VALVE DEVICE
APPAREIL À ONDES DE CHOC POURVU DE DISPOSITIF À DOUBLE SOUPAPE

(43) Veröffentlichungstag der Anmeldung: 28.02.2024
(73) Patentinhaber: STORZ MEDICAL AG, 8274 Tägerwilen (CH)
(72) Erfinder: STORZ, Rafael, 8280 Kreuzlingen (CH); BELAU, Markus, 78467 Konstanz (DE); KÜHL, Arvid, 8274 Tägerwilen (CH); HONSELL, Lukas, 78479 Reichenau (DE); GREMLICH, Felix, 8280 Kreuzlingen (CH); GLENZER, Thomas, 8280 Kreuzlingen (CH)
(74) Vertreter: Szynka Smorodin Patentanwälte Partnerschaft mbB

(56) Entgegenhaltungen:
- EP-A1- 2 529 679
- EP-B1- 2 213 273
- US-A1- 2009 326 425
- US-A1- 2011 275 965
- US-A1- 2014 350 438

## Beschreibung

Die Erfindung bezieht sich auf ein Gerät zur Behandlung des menschlichen oder tierischen Körpers mit mechanischen Druckwellen, welche durch Aufschlagen eines beschleunigten Projektils auf einen Applikator erzeugt werden.

Geräte dieses Typs sind seit einiger Zeit bekannt und zunehmend im Einsatz. Zur Behandlung des (menschlichen oder tierischen) Patienten dienen mechanische Druckwellen, die durch Auflegen eines Applikators auf den Körper des Patienten eingekoppelt werden und durch eine Kollision eines beschleunigten Projektils mit dem Applikator erzeugt werden. Dabei muss der Applikator nicht zwingend einstückig sein, sondern kann auch aus einer Mehrzahl verschiedener Teile oder Materialien zusammengesetzt sein.

Eine in der Praxis bewährte und vielfach beschriebene Technik zur Beschleunigung des Projektils ist pneumatisch. Dabei wird durch Beaufschlagung eines Volumens auf einer Seite des entlang einer Bewegungsstrecke, z. B. in einem Rohrstück, beweglichen Projektils ein pneumatischer Überdruck eingekoppelt.

Im Stand der Technik wird hierfür ein Schaltventil benutzt, das an eine Pneumatikversorgung, insbesondere einen Kompressor mit regelbarem Ausgangsdruck, angeschlossen ist und dessen Puls das Projektil von einem zu dem Applikator distalen Ende der Bewegungsstrecke aus hin zu dem Applikator beschleunigt. Die pneumatische Beaufschlagung wird bei Erreichen des proximalen Endes der Bewegungsstrecke ausgeschaltet, also mit dem Aufschlagen auf dem Applikator.

Die Rückbewegung erfolgt im Stand der Technik mithilfe einer Gegendruckkammer, also eines Speichervolumens, in das das zu dem Applikator hin bewegte Projektil gewissermaßen die vor ihm befindliche Luft verdrängt, womit es dieses Speichervolumen quasi aufpumpt.

In der vorveröffentlichten, allerdings wegen mangelnder Ausführbarkeit im Einspruchsbeschwerdeverfahren widerrufenen EP 2 181 730 B1 wird neben einer nicht näher ausgeführten Steuerung der Öffnungszeit des Schaltventils für die Beschleunigung auch eine gezielte Druckbegrenzung in dieser Gegendruckkammer diskutiert. Außerdem erwähnt dieses Dokument den Einsatz eines zweiten Schaltventils für eine Rückführung des Projektils in die distale Ausgangslage nach der Beaufschlagung durch das erste Schaltventil.

Die US 2014/350438 A1 beschreibt ein ballistisches Druckwellengerät nach dem Oberbegriff des Anspruchs 1, das zur Erzeugung besonders intensiver Wellen zur Zerkleinerung von Körperkonkrementen ausgelegt ist. Zur Rückführung des Projektils wird unter anderem eine pneumatische Lösung mit einem zweiten Ventil vorgeschlagen.

Die US 2009/326425 A1 beschreibt ein ballistisches Druckwellengerät, dessen pneumatisches System dazu ausgelegt ist, dass in Folge eines Beschleunigungsvorgangs des Projektils mindestens zwei Kollisionen stattfinden.

Die DE 20 2010 009 899 U1 beschreibt ein elektromagnetisches Druckwellengerät, das zur Rückführung des Projektils und Vermeidung von Schäden einer Rückstellfeder einen zweiten Elektromagneten vorschlägt.

Der vorliegenden Erfindung liegt die Aufgabe zugrunde, auf dieser Grundlage ein hinsichtlich der Hin- und Rückbewegung des Projektils verbessertes Gerät des beschriebenen Typs mit pneumatischer Einrichtung zur Projektilbewegung anzugeben.

Zur Lösung dieser Aufgabe wird das Gerät gemäß Anspruch 1 vorgeschlagen. Bevorzugte Ausgestaltungen sind Gegenstand der abhängigen Ansprüche.

Dementsprechend weist das erfindungsgemäße Gerät als Teil seiner pneumatischen Einrichtung eine Doppelventileinrichtung zur Beaufschlagung des Projektils in beide Richtungen, also zu dem Applikator hin und umgekehrt von ihm weg in der Rückrichtung, auf, also z. B. die Kombination eines ersten und eines zweiten Ventils. Die Zeitphasen, in denen das Projektil so pneumatisch beaufschlagt wird, dass es sich in der Hinrichtung bewegt, also z. B. die Einschaltphase eines ersten Ventils, wird im Folgenden als erste Einschaltzeit bezeichnet und umgekehrt als zweite Einschaltzeit eine Zeitphase einer umgekehrten Beaufschlagung des Projektils. Erfindungsgemäß soll das Gerät dazu ausgelegt sein (also insbesondere eine darin vorhandene Steuereinrichtung so ausgelegt sein), dass die zweite Einschaltzeit variabel ist. Dies kann zusätzlich zu einer Variabilität der ersten Einschaltzeit oder auch bei einer fest vorgegeben ersten Einschaltzeit der Fall sein.

Je nach Anforderungen und Schwerpunkten im Einzelfall lassen sich damit verschiedene Vorteile erzielen. Insbesondere kann die Aufschlaggeschwindigkeit des Projektils auf dem Applikator verändert werden, und zwar auch unabhängig von einer Veränderung des beschleunigenden Drucks. Z. B. kann die zweite Einschaltzeit schon vor der Kollision zwischen dem Projektil und dem Applikator beginnen, wobei dieser Teil vor der Kollision variabel ist. Wenn nämlich dieser Teil der zweiten Einschaltzeit erstens in einer Schlussphase der ersten Einschaltzeit mit dieser überlappt und/oder zweitens nach dem Ende der ersten Einschaltzeit, aber vor der Kollision liegt, so verringert sich die Aufschlaggeschwindigkeit.

Bei einer Überlappung beider Einschaltzeiten könnte bei beispielsweise ungefähr gleichem pneumatischen Druck auf beiden Seiten des Projektils eine Kompensation der in der Hinrichtung beschleunigenden Kraft erfolgen und das Projektil in dieser Zeitphase ("Überlappzeit") damit quasi kräftefrei oder jedenfalls mit reduzierter beschleunigender Kraft bewegt werden. In dem anderen Fall (ohne Überlappung) würde das Projektil tatsächlich abgebremst. Für beide Fälle gilt, dass sich ohne Verringerung des Drucks verringerte Aufschlaggeschwindigkeiten realisieren lassen.

Zum einen ist das ein weiterer Freiheitsgrad bei der Steuerung. Zum anderen könnte (zusätzlich oder unabhängig davon) ein relativ großer Druck und damit eine große mittlere Geschwindigkeit bei der Rückführung des Projektils ohne diesem Druck entsprechende hohe Aufschlaggeschwindigkeiten (bei angenommener Beschleunigung des Projektils durch diesen Druck in Hinrichtung) ermöglicht werden.

Zusätzlich oder unabhängig davon kann aber auch die Rückführung des Projektils mit unterschiedlichen Beschleunigungen infolge eines unterschiedlich großen Anteils der zweiten Einschaltzeit nach der Kollision (einschließlich des Falls, dass diese ausschließlich nach der Kollision vorliegt) realisiert werden. Damit lässt sich auch die Rückführung ohne Veränderung des Drucks beeinflussen, und zwar hinsichtlich Zeitdauer und Geschwindigkeit. Das kann z. B. bei einem relativ großen anliegenden pneumatischen Druck von Interesse sein.

Die zweite Einschaltzeit kann auch über den Neubeginn der nächsten Bewegung des Projektils in der Hinrichtung zu dem Applikator hinaus andauern. Mit einem über den Neubeginn der Projektilbewegung in der Hinrichtung hinaus andauernden Teil einer zweiten Einschaltzeit lässt sich jedenfalls auch die Aufschlaggeschwindigkeit bei der nächsten Kollision steuern, nämlich indem dieser in die Hinbewegung fallende Anteil der zweiten Einschaltzeit variiert wird. Prinzipiell kann auch die Rückbewegung durch eine zweite Einschaltzeit begonnen werden und dann nach deren Ende eine weitere zweite Einschaltzeit gegen Ende dieser Rückbewegung einsetzen.

Zusätzlich oder unabhängig davon kann natürlich im weiteren Verlauf vor dem Ende dieser Hinbewegung des Projektils eine neue zweite Einschaltzeit beginnen und ihrerseits zusätzlich oder alternativ zur Steuerung der Aufschlaggeschwindigkeit genutzt werden.

Aus den obigen Erläuterungen ergibt sich bereits, dass die Variabilität der zweiten Einschaltzeit ihre zeitliche Lage (relativ zu dem Aufschlagen des Projektils auf dem Applikator) und/oder ihre zeitliche Dauer betreffen kann. Z. B. könnte die selbe zweite Einschaltzeit eine konstante Dauer nach dem Aufschlagen, aber eine variable Dauer vor dem Aufschlagen und damit einen variablen Beginn und eine variable Gesamtdauer haben oder umgekehrt einen festen Beginn (relativ zu dem Aufschlagen) und ein variables Ende. Außerdem könnte sie eine feste Dauer haben, könnten aber dabei Beginn und Ende variieren, und natürlich könnten Beginn, Ende und Dauer variieren.

Weiter oben wurde die Kombination zweier Schaltventile angesprochen, die eine Möglichkeit für eine erfindungsgemäß vorgesehene Doppelventileinrichtung darstellt. Bei dieser Variante sind die beiden Ventile (vorzugsweise unabhängig voneinander) von der Steuereinrichtung ansteuerbar. Alternativ kann aber auch ein einheitliches Ventil verwendet werden, das hier als "Kombinationsventil" bezeichnet wird und das abhängig von der Ansteuerung durch die Steuereinrichtung mindestens zwei Schaltzustände aufweist, nämlich einen ersten zur Beaufschlagung des Projektils in Richtung zu dem Applikator hin und einen zweiten zur Beaufschlagung in der Rückrichtung. Während das Kombinationsventil in dem ersten Schaltzustand ist, ist also eine erste Ventilöffnungszeit gegeben, und im zweiten Schaltzustand dementsprechend eine zweite Ventilöffnungszeit.

In diesen beiden Schaltzuständen wird der jeweils im anderen Schaltzustand zu beaufschlagende pneumatische Anschluss durch das Kombinationsventil vorzugsweise belüftet, sodass also z. B. bei der Hinbewegung auf der zu dem Applikator proximalen Seite des Projektils ungefähr Umgebungsdruck herrscht und im Unterschied zu dem konventionellen Vorgehen mit einer Gegendruckkammer kein von Kollision zu Kollision zunehmender Staudruck.

Das Kombinationsventil hat optional noch einen weiteren dritten Schaltzustand, in welchem die beiden pneumatischen Anschlüsse (gleichzeitig) mit dem pneumatischen Versorgungsdruck beaufschlagt werden. In diesem dritten Schaltzustand ist also der Überlapp zwischen der ersten Einschaltzeit und der zweiten Einschaltzeit gegeben. Wenn also von dem Beginn der zweiten Einschaltzeit während der ersten Einschaltzeit (oder umgekehrt) die Rede ist, bedeutet dies bei der Variante mit dem Kombinationsventil ein Umschalten dieses Kombinationsventils. Das Gleiche gilt für ein Ende der ersten Einschaltzeit während noch bestehender zweiter Einschaltzeit (oder umgekehrt).

Auch bei dem Einsatz zweier separater Ventile ist vorzugsweise mindestens eines der beiden Ventile ein "Zweiwegeventil", das dementsprechend eine Belüftung durchführt, sofern es nicht zur Beaufschlagung mit dem pneumatischen Druck geschaltet ist. Weitere Schaltzustände sind allerdings nicht ausgeschlossen und das Ventil ist nicht zwingend auf genau zwei Schaltzustände begrenzt.

Mit einer Belüftung ist im Übrigen eine pneumatisch gut leitfähige Verbindung zur Außenatmosphäre oder einem dieser im Wesentlichen entsprechenden Referenzdruckvolumen gemeint. Es geht also nicht um eine bewusste Verzögerung des Abfließens von Gas unter Überdruck im Sinn einer Drosselung.

Alternativ zu einer Belüftung über das Kombinationsventil oder die gerade angesprochenen Zweiwegeventile könnte das Gerät auch z. B. eine gewisse pneumatische Undichtigkeit haben und in Abwesenheit einer Beaufschlagung mit pneumatischem Druck so oder anders quasi kriechend selbst eine gedrosselte Entlüftung durchführen. Diese Möglichkeit ist aber weniger bevorzugt.

Vorzugsweise wird die Länge der zweiten Einschaltzeit variiert. Vorzugsweise wird dabei das zeitliche Ende der zweiten Einschaltzeit, und zwar von der Kollision aus gemessen, beim Steuern konstant gehalten und wird dementsprechend nur der Anfangszeitpunkt der zweiten Einschaltzeit beim Steuern variiert, z. B. weil die Rückführung des Projektils im Wesentlichen gleich erfolgt.

Neben der bereits angesprochenen Möglichkeit einer Überlappzeit zwischen der ersten und der zweiten Einschaltzeit kommt außerdem gewissermaßen das Gegenteil in Betracht, also eine Abstandszeit zwischen dem Ende der ersten und dem Beginn der zweiten Einschaltzeit. Auch diese kann (muss aber nicht) variabel sein. Z. B. könnte eine solche Abstandszeit vor dem Aufschlagen des Projektils auf dem Applikator liegen, wobei das Projektil während der ersten Einschaltzeit beschleunigt wird, während der Abstandszeit weitgehend (und von Reibung abgesehen) kräftefrei "weiterfliegt" und dann infolge eines Gegendrucks während der zweiten Einschaltzeit vor dem Aufschlagen bereits etwas abgebremst wird. Ähnliches gilt für einen Beginn der zweiten Einschaltzeit mit oder nach dem Aufschlagen. Insbesondere können die Fälle auch "gemischt" auftreten, kann es also Steuerungszustände geben mit Überlappzeit (einschließlich Null) und solche mit Abstandszeit (auch einschließlich Null, was einer Überlappzeit Null gleichkommt).

Die pneumatische Einrichtung kann im einfachsten Fall einen Anschluss zur Versorgung aus einem pneumatischen Leitungsnetz, z. B. in einem Krankenhaus, oder aus einer Druckgasflasche aufweisen. Bevorzugt ist jedoch ein Pneumatikkompressor, mit dem das erfindungsgemäße Gerät örtlich unabhängig und im Vergleich zu einer Druckgasflasche mobiler ist. An sich sind Pneumatikkompressoren im Zusammenhang mit solchen Geräten vorbekannt. Die Erfindung bietet jedoch den besonderen Aspekt, bei verschiedenen Steuerungszuständen mit unterschiedlichen Aufschlaggeschwindigkeiten des Projektils nicht zwingend den Versorgungsdruck ändern zu müssen. In anderen Worten kann der Kompressor in solchen verschiedenen Steuerungszuständen bei gleicher Drehzahl laufen.

Natürlich kann dadurch zunächst einmal die Ansteuerung des Kompressors vereinfacht werden, insbesondere wenn dieser grundsätzlich im eingeschalteten Zustand immer bei der gleichen Drehzahl läuft. Ferner kann der Kompressor dabei in der Nähe seines oder bei seinem Effizienzmaximum (hinsichtlich der Drehzahl) betrieben werden. Überdies ist es möglich, Geräuschminderungsmaßnahmen, z. B. eine dämpfende Lagerung des Kompressors oder eine geräuschdämmende Umhüllung, auf das Schwingungsverhalten des Kompressors bei der einen gleichen Drehzahl abzustimmen.

Eine besondere Gestaltungsmöglichkeit der Erfindung liegt darin begründet, allein durch Veränderung von Ventilöffnungszeitpunkten oder Ventilöffnungszeitdauern die Stoßphysik zwischen Projektil und Applikator direkt und schnell beeinflussen zu können, insbesondere die Aufschlaggeschwindigkeit und damit den Impuls beim Aufschlag. Im Vergleich zu einer Veränderung des Versorgungsdrucks ist diese Einflussnahmemöglichkeit besonders schnell, sodass in einem iterativen Betriebszustand prinzipiell von einem Aufschlagprozess zum nächsten die Aufschlaggeschwindigkeit/-impuls der kombinierten Hin- und Rückbewegung verändert werden können. Eine so schnelle und freie Einflussnahme erlaubt der Stand der Technik nicht.

Typische Aufschlaggeschwindigkeiten liegen dabei, aber auch bei sich weniger schnell oder nicht verändernden Bedingungen, im Bereich zwischen 2 m/s und 30 m/s. Für die Stoßphysik ist vor allem der Aufschlagimpuls wichtig, der bei typischen Projektilmassen zwischen 1 g und 10 g, vorzugsweise zwischen 2 g und 5 g, somit in einem Bereich von 2 gm/s bis 300 gm/s, vorzugsweise zwischen 10 gm/s und 150 gm/s liegen kann.

Bei einer besonderen Ausgestaltung weist das Gerät eine Messeinrichtung auf, mit der der Durchtritt des Projektils an einer Stelle seiner Bewegungsstrecke gemessen werden kann. Diese Messeinrichtung kann mit der Steuereinrichtung gekoppelt sein. Damit kann in solcher Form z. B der Durchtritt des Projektils kurz vor dem Aufschlagen oder quasi beim Aufschlagen auf den Applikator erfasst werden, sodass die Einschaltzeiten dementsprechend (insbesondere hinsichtlich ihres Anfangs und ihres Endes) auf den Endzeitpunkt des Aufschlagens abgestimmt werden können.

Eine solche Erfassung kann z. B. optisch erfolgen, etwa durch eine Lichtschranke oder dergleichen, vorzugsweise aber induktiv unter Verwendung einer Messspule. Diese kann durch einen Restmagnetismus des Projektils oder rein induktiv (durch Veränderung der Streuinduktivität) das Projektil erfassen.

Im Folgenden wird die Erfindung anhand von Ausführungsbeispielen näher erläutert, wobei die einzelnen Merkmale im Rahmen der Ansprüche auch in anderer Kombination erfindungswesentlich sein können.

Im Einzelnen zeigt
- Figur 1: eine perspektivische Darstellung eines erfindungsgemäßen Geräts, wobei ein mittlerer Gehäuseteil der Übersichtlichkeit halber weggelassen ist;
- Figur 2: einen Längsschnitt durch das Gerät aus Figur 1 in gegenüber Figur 1 rechts-links-vertauschter Lage;
- Figur 3: ein schematisches Diagramm des Handstücks mit einem zugehörigen Basisgerät;
- Figur 4: eine Folge schematischer Zeitverlaufsdiagramme 4a) bis e) zur Erläuterung der Funktionsweise;
- Figur 5: eine schematische Darstellung eines Kombinationsventils zur Erläuterung eines alternativen Ausführungsbeispiels zu den Figuren 1 und 2;
- Figur 6: eine Folge schematischer Zeitverlauf Diagramme 6a) bis f) zur Erläuterung weiterer Steuerungszustände in Ergänzung zu Figur 4;
- Figur 7: ein weiteres schematisches Verlaufsdiagramm zur Erläuterung der periodischen Arbeitsweise;
- Figur 8: eine Folge schematischer Zeitverlaufsdiagramme 8a) bis c) zur weiteren Erläuterung der Funktionsweise;
- Figur 9: eine wiederkehrende Folge von zwei verschiedenen Projektilgeschwindigkeitsniveaus in direkter Abfolge, wobei nach einem Puls mit hoher Geschwindigkeit zwei Pulse mit niedriger Geschwindigkeit direkt nachfolgen;
- Figur 10: eine Steuersequenz für die Ansteuerung der Ventile V1 und V2 gemäss der in Figur 9 gezeigten Folge von zwei verschiedenen Projektilgeschwindigkeitsniveaus;
- Figur 11: eine höhere zeitliche Detaillierung der ersten 300 ms aus der Steuersequenz von Figur 10.

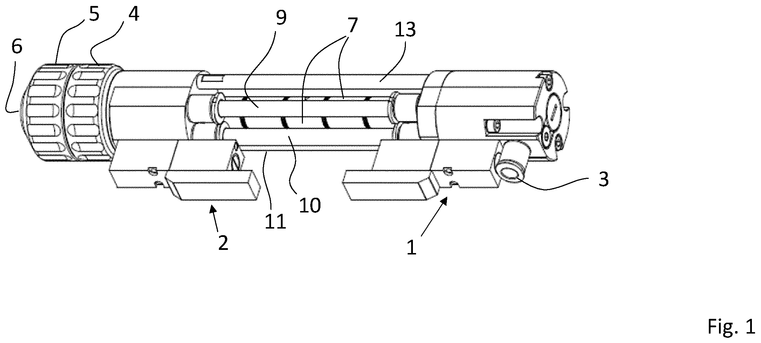

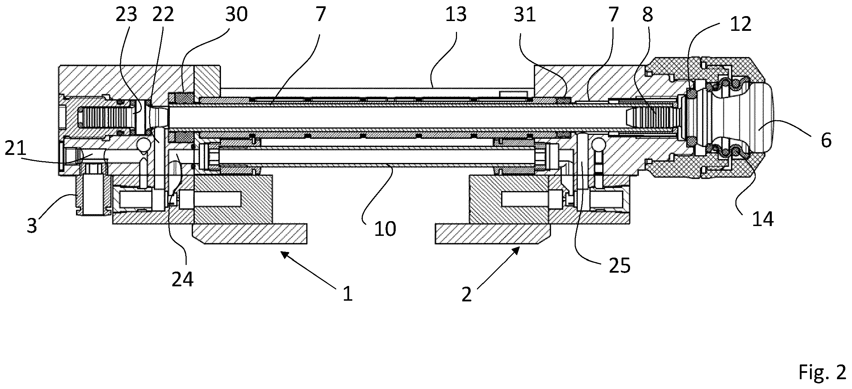

Figur 1 zeigt ein Handstück eines erfindungsgemäßen Geräts in perspektivischer Ansicht mit nach vorn-links weisenden pneumatischen Ventilen, nämlich einem ersten Ventil 1 und einem zweiten Ventil 2. Rechts sieht man einen pneumatischen Versorgungsanschluss 3 und links zwei zur leichteren Handhabung jeweils außen geriffelte Schraubringe 4 und 5 zur Halterung des im Folgenden noch näher erläuterten Applikators 6. Dieser ist in Figur 1 ganz links mit seiner patientenzugewandten Oberfläche gerade noch zu sehen und im Übrigen in Figur 2 gezeigt. Er könnte auch mehrteilig aufgebaut sein.

Im mittleren Bereich des Geräts aus Figur 1 erkennt man eine Mehrzahl in Querrichtung laufender Röhren, wobei die mittlere mit dem Bezugszeichen 7 das in Figur 2 im Schnitt erkennbare Projektil 8 enthält und führt. Davor sieht man zwei parallele pneumatische Verbindungsrohrleitungen 9 und 10 zwischen den beiden Ventilen 1 und 2, wobei die Rohrleitung 9 zum Zuführen einer Druckbeaufschlagung zu dem zweiten Ventil 2 und die Rohrleitung 10 umgekehrt zur Entlüftung dieses zweiten Ventils 2 über einen bei dem ersten Ventil 1 vorgesehenen Auslass dient. Diese Mehrzahl Rohre ist bei diesem Ausführungsbeispiel von einer in Figur 1 durch die Linie unter der Rohrleitung 10 und die beiden Linien über dem Projektilführungsrohr 7 dargestellten Gehäuseabdeckung 11 umgeben. Diese Gehäuseabdeckung 11 läuft in Figur 1 im hinteren Bereich und umfasst nur einen Teil des Umfangs. Sie ist an ihren jeweiligen axialen Kanten ähnlich einer Bördelung durch ein verrundetes Umschlagen nach innen griffgünstig gestaltet, was in Figur 1 bei der oberen Kante angedeutet ist. Die Gehäuseabdeckung 11 kann also bei der praktischen Handhabung als Griff dienen. Der Abstandshalter 13 stabilisiert den Aufbau und verbindet die beiden Enden des Handstücks mechanisch.

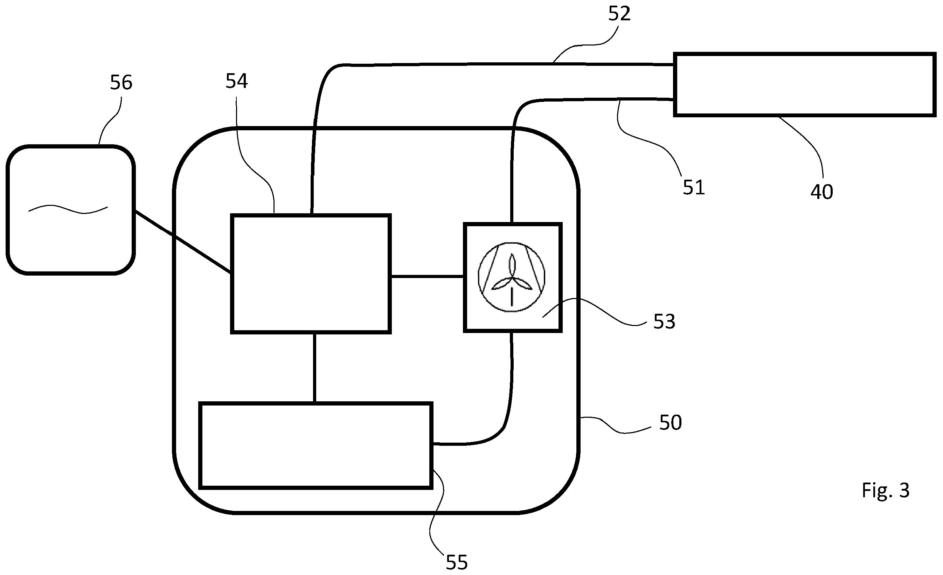

Eine von einem Pneumatikkompressor zu dem Gerät führende flexible Druckluftzuleitung (vgl. 51 in Figur 3) ist hier nicht eingezeichnet und an den bereits erwähnten Anschluss 3 anzuschließen. Analog ist eine elektronische Steuerleitung (52 in Figur 3) von einer externen Steuerung zu den Ventilen 1 und 2 nicht eingezeichnet, die mit der Druckluftzuleitung einheitlich ausgeführt sein kann.

Figur 2 zeigt einen Längsschnitt entlang einer gedachten Mittellängsachse der bereits erwähnten Zylinderform des Gesamtgeräts, die gleichzeitig eine Mittellängsachse des Projektilführungsrohrs 7 ist. Zur Veranschaulichung der Dimensionen: Die Länge des Projektilführungsrohrs 7 beträgt bei diesem Ausführungsbeispiel 145.5 mm und die übrige Darstellung in Figur 2 ist maßstäblich. In diesem Projektilführungsrohr ist das Projektil 8 in Figur 2 rechts eingezeichnet und damit in Anlage an dem Applikator 6, der von dem beschriebenen Schraubring 4 und 5 in einer an sich bekannten Weise gehalten ist. Dabei ist der Applikator 6 mit einem balgartigen Elastomerring 14 in Axialrichtung elastisch gelagert und mit einem weiteren Elastomerring 12 pneumatisch abgedichtet. Alternativ ist auch ein Geräteaufbau hinsichtlich des Applikators 6 und seiner Halterung und Abdichtung gemäß z. B. der EP 2 529 679 (auch unabhängig von der dort gezeigten Kappe) oder der EP 2 095 843 (auch unabhängig von dem dort thematisierten Material Keramik) möglich und bevorzugt.

Figur 2 zeigt links einen inneren Kanal 21, der den Pneumatikanschluss 3 mit dem ersten Ventil 1 verbindet. Das erste Ventil 1 kann demzufolge einen an dem Pneumatikanschluss 3 anstehenden Versorgungsdruck steuerungsabhängig auf einen radialen Kanal 22 schalten, der unter einem Dämpferelement 23 mündet und damit an das Innenvolumen des Projektilführungsrohrs 7 angeschlossen ist. Über diesen Kanal 22 wird also das Projektil während einer ersten Einschaltzeit in Richtung zu dem Applikator 6 hin beaufschlagt bzw. beschleunigt. Unabhängig davon wird der pneumatische Versorgungsdruck über den Kanal 24 und das Rohr 10 an das zweite Ventil 2 weitergegeben.

In der zweiten alternativen Schaltstellung werden der Kanal 22 und damit auch das Innenvolumen des Projektilführungsrohrs 7 zwischen dem distalen Ende (in Figur 2 links) und dem Projektil 8 belüftet.

In dem zweiten Ventil 2, das grundsätzlich spiegelsymmetrisch zu dem ersten Ventil 1 aufgebaut ist, kann der über das Rohr 10 anliegende pneumatische Versorgungsdruck alternativ über den Kanal 25 radial nach oben gegeben werden zu einem das Projektilführungsrohr 7 umgebenden Volumen (in Figur 2 als Schlitz über und unter dem Rohr 7 zu erkennen), das von dem Anschluss des Kanals 25 aus nach rechts, also in Richtung zu dem Applikator 6, führt und dort zwischen dem Applikator 6 und dem zu ihm proximalen Ende des Projektilführungsrohrs 7 an das Innenvolumen des Projektilführungsrohrs 7 angeschlossen ist (von der in Figur 2 gezeichneten Anwesenheit des Projektils 8 dort abgesehen). Über den Kanal 25 kann also der pneumatische Versorgungsdruck schaltbar an das Innenvolumen des Projektilführungsrohrs 7 zwischen dem Applikator 6 und dem Projektil 8 angelegt werden. Dabei ist allerdings bei diesem Beispiel die pneumatische Verbindung in Folge eines kleineren effektiven Öffnungsquerschnitts etwas schlechter, als an der entgegengesetzten Seite des Projektilführungsrohrs 7, so dass sich hier bei größeren Luftströmungsgeschwindigkeiten (höhere Frequenzen, größere Drücke) früher oder stärker Verzögerungen bemerkbar machen.

Alternativ kann das zweite Ventil 2 in der anderen Schaltstellung den Anschluss des Innenvolumens des Rohrs 10 an ihm sperren und den Kanal 25 und damit das Innenvolumen des Projektilführungsrohrs 7 rechts vom Projektil 8 belüften, also über eine pneumatisch gut leitfähige Verbindung an die Außenatmosphäre anschließen.

Die beiden Ventile 1 und 2 können mithin das Projektil von beiden Seiten mit pneumatischem Druck beaufschlagen, und zwar unabhängig voneinander und damit gleichzeitig oder wechselseitig oder können das Innere des Projektilführungsrohrs 7 beiderseits belüften.

Das Bezugszeichen 30 in Figur 2 bezeichnet einen ringförmigen Permanentmagneten am gegenüber dem Applikator 6 distalen Ende der (mit der Länge des Projektilführungsrohrs 7 zusammenfallenden) Bewegungsstrecke des Projektils 8. Mit diesem Magneten 30 kann das aus ferromagnetischem Material aufgebaute Projektil 8 an diesem distalen Ende der Bewegungsstrecke leicht fixiert werden. Durch einseitige Druckbeaufschlagung mittels des Ventils 2 kann das Projektil ferner in diese Position zurückgeführt und optional auch zusätzlich dort gehalten werden, insbesondere bei Betriebsbeginn oder bei nicht ferromagnetischem Projektil. Insoweit kann der Permanentmagnet 30 optional auch weggelassen werden, vor allem dann, wenn die im weiteren Verlauf noch erläuterten Reflexionen an diesem distalen Ende der Bewegungsstrecke auch bei kleinen Aufprallgeschwindigkeiten des Projektils 8 dort ermöglicht werden sollen.

Mit 31 ist eine Stelle beziffert, an der man mit einer Messspule den Durchtritt des Projektils 8 durch die entsprechende Stelle der Bewegungsstrecke erfassen könnte, wobei diese Stelle relativ nah an dem Applikator 6 liegt. Im einfachsten Fall nutzt man hier einen leichten Restmagnetismus des Projektils 8 aus, aber man könnte natürlich auch die Veränderung der Induktivität der Spule 31 wechselstromtechnisch erfassen und auswerten. Man kann die Kollision des Projektils 8 mit dem Applikator 6 auch durch die Verwendung eines Mikrophons oder Bewegungssensors im Versuchsaufbau ermitteln. Außerdem kann man im Versuchsaufbau z. B. mit zwei kurz vor dem Applikator 6 positionierten Lichtschranken die Aufprallgeschwindigkeit des Projektils 8 ermitteln.

Figur 3 zeigt ein Blockschema mit dem in den Figuren 1 und 2 dargestellten Gerät rechts oben, und zwar mit dem Bezugszeichen 40 summarisch bezeichnet. Dies Gerät 40 ist ein in der Hand zu haltendes mobiles Handstück, wie es von einschlägigen Geräten aus dem Stand der Technik an sich bereits bekannt ist. Es ist über zwei Leitungen 51 und 52 an eine Basisstation 50 angeschlossen, welche einen Pneumatikkompressor 53 und eine Steuerung 54 enthält. Der Kompressor 53 ist über die Leitung 51, nämlich eine pneumatische flexible Schlauchleitung, mit dem Handgerät 40 verbunden und die Steuerung 54 über die (optional mit der Leitung 51 integrierte) elektrische Leitung 52, über die die Steuerung auf die bereits erwähnten beiden Ventile 1 und 2 zugreifen und diese mit Strom versorgen kann. Über die Leitung 52 kann außerdem eine Kommunikation mit dem Handstück 40 erfolgen, insbesondere wenn dort zusätzlich eine Steuerung bzw. ein Teil der Steuerung vorgesehen ist.

Die Steuerung 54 steuert im Übrigen auch den Kompressor 53 hinsichtlich seiner Drehzahl und natürlich des Ein- und Ausschaltens und wird ihrerseits, genauso wie der Kompressor 53, von einem Netzgerät 55 leistungsversorgt. In dem Kompressor 53 kann aber auch eine die Drehzahl beeinflussende Druckregelung oder ein Regelventil integriert sein. Außerdem ist die Steuerung 54 an ein Display 56 angeschlossen, das in dem Basisgerät 50 eingebaut oder auch separat davon implementiert sein kann. Das Basisgerät 50 wird über ein berührungsempfindliches Display 56 und/oder über eine hier nicht dargestellte Anordnung von Tasten bedient.

Der Benutzer kann also die Funktion des Geräts 40 anhand solcher Tasten und jedenfalls anhand des Displays 56 steuern, wobei die Steuerung 54 insbesondere die Öffnungs- und Schließzeiten und damit auch die Öffnungsdauern der beiden Ventile 1 und 2 vorgibt. Teilaufgaben der Steuerung 54 können auch im Handstück 40 integriert sein, besonders was die Ansteuerung der Ventile 1 und 2 betrifft.

Zum Grundverständnis der Ansteuerung der beiden Ventile kann verwiesen werden auf das ältere Patent EP 2 213 273 B1. Das Ausführungsbeispiel darin entspricht hinsichtlich der Dimensionierung insbesondere des Projektilführungsrohrs und des Projektils weitgehend den obigen Erläuterungen und den Figuren 1 und 2 mit Ausnahme der Existenz des zweiten Ventils 2 und des Wegfalls der Gegendruckkammer. Außerdem wird in dem zitierten Ausführungsbeispiel von einer bestimmten Ventilöffnungszeit des dort einzigen Ventils bei einem bestimmten Druck ausgegangen, wohingegen die Projektilbeschleunigung im vorliegenden Fall durch den Anteil der ersten Ventilöffnungszeit auch außerhalb der Überlappzeit und damit auch bei einem konstanten Druck variabel erfolgt. Für die folgenden Erläuterungen kann beispielhaft von einem Druck von 4 bar ausgegangen werden. Damit ergibt sich folgende beispielhafte Wertetabelle mit gemessenen Werten:
Wertetabelle

| | | | | | |
|---|---|---|---|---|---|
| Projektilgeschwindigkeit [m/s] | 10 | 12 | 14 | 16 | 18 |
| Öffnungszeitpunkt Ventil 1 [ms] | 0 | 0 | 0 | 0 | 0 |
| Schließzeitpunkt Ventil 1 [ms] | 13 | 13 | 13 | 13 | 13 |
| Öffnungszeitpunkt Ventil 2 [ms] | 2.6 | 3 | 3.7 | 5 | 7.1 |
| Schließzeitpunkt Ventil 2 [ms] | 18 | 18 | 18 | 18 | 18 |
| Einschaltzeitdauer Ventil 2 [ms] | 15.4 | 15 | 14.3 | 13 | 10.9 |
| Überlappzeit [ms] | 10.4 | 10 | 9.3 | 8 | 5.9 |
| Aufprallzeitpunkt [ms] | 19.4 | 18.7 | 18.0 | 17.3 | 16.6 |

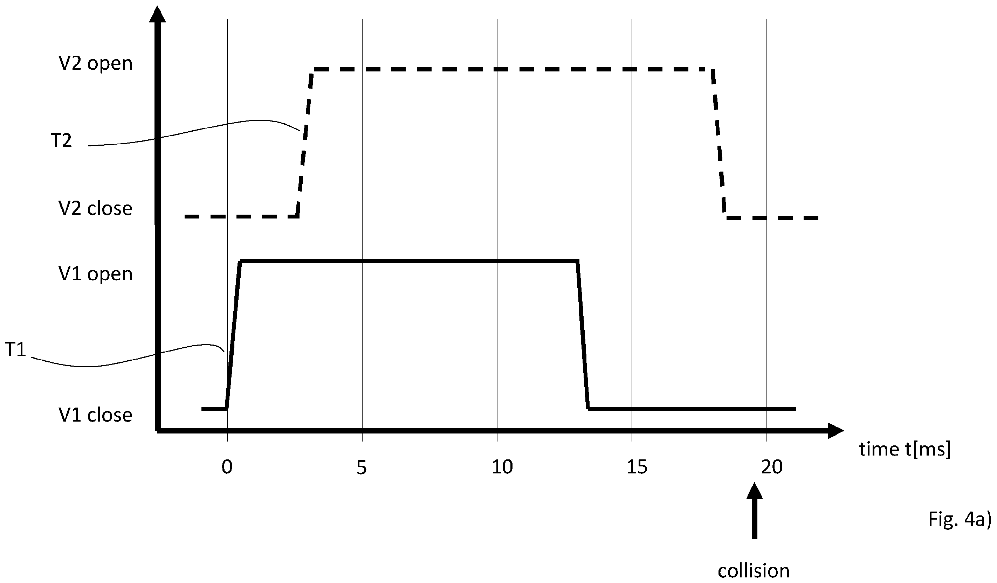

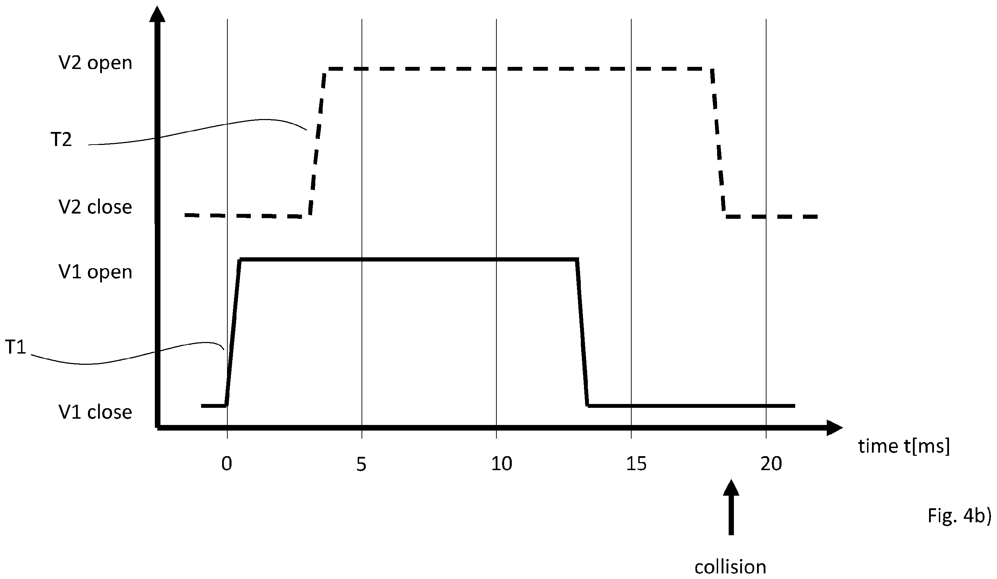

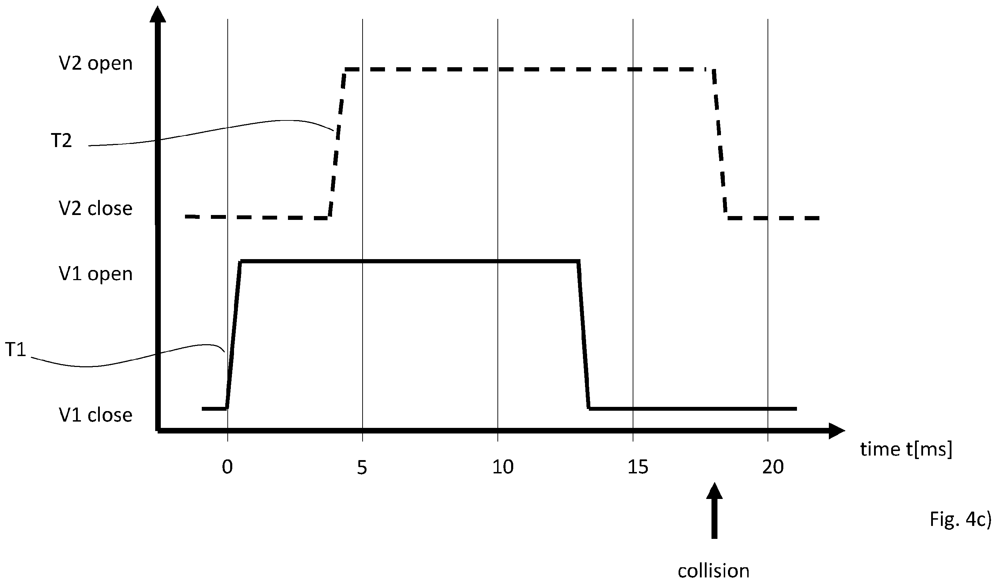

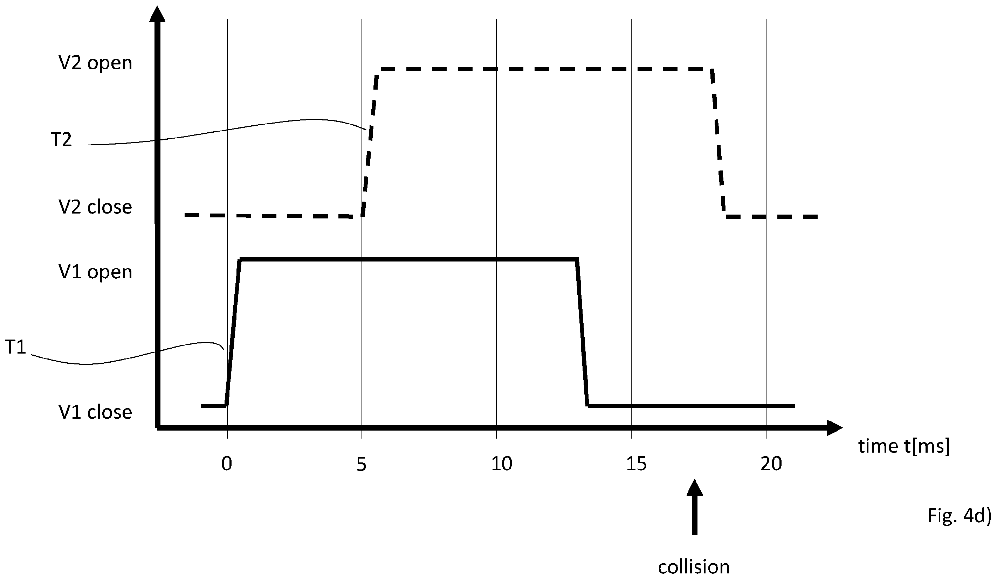

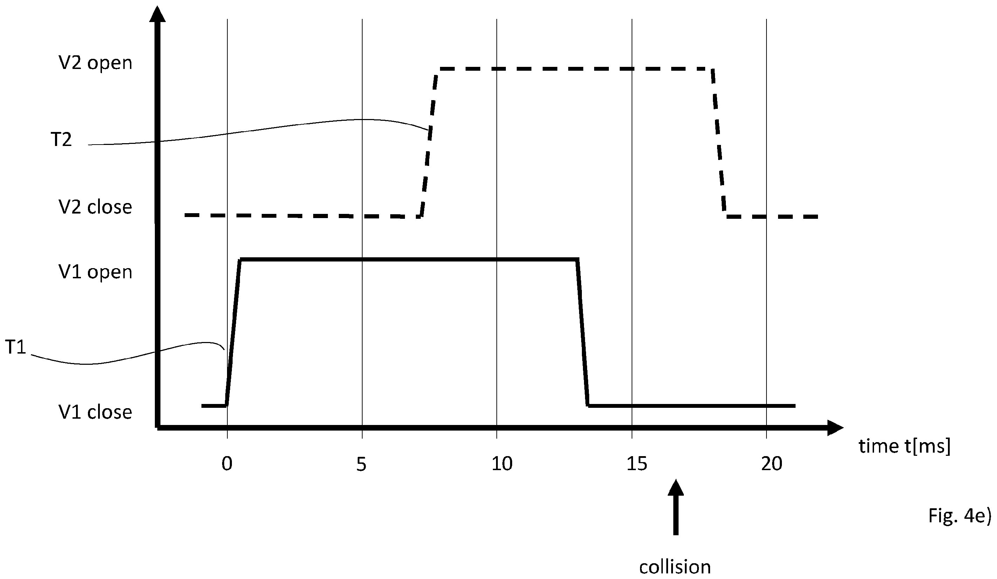

Figur 4 zeigt eine Folge von fünf einzelnen schematischen Zeitverlaufsdiagrammen 4a) bis 4e) entsprechend der obigen Tabelle, in denen jeweils mit der mit T1 bezeichneten Kurve der Öffnungs- und Schließvorgang des ersten Ventils 1 und mit der mit T2 bezeichneten Kurve analog der Öffnungs- und Schließvorgang des zweiten Ventils T2 bezeichnet ist. Der erhöhte Kurventeil entspricht also jeweils der ersten/zweiten Einschaltzeit.

Im Vergleich erkennt man, dass die erste Einschaltzeit bei allen fünf Steuerungszuständen auf der (willkürlichen) Zeitachse in der horizontalen bei 0 ms beginnt und bei 13 ms endet. Demgegenüber verschiebt sich die zweite Einschaltzeit hinsichtlich ihres Beginns von anfangs 2,6 ms in Figur 4a) schrittweise bis 7,1 ms in Figur 4e), wohingegen die zweite Einschaltzeit in allen fünf Darstellungen bei 18 ms endet. Die zweite Einschaltzeit ist also hinsichtlich ihres Beginns und ihrer Dauer variabel. Ferner gibt es in allen Steuerungszuständen eine Überlappzeit, nämlich von etwa 3 ms bis 13 ms in Figur 4a) bis hin zu noch von etwa 7 ms bis 13 ms in Figur 4e), wobei diese Überlappzeit schrittweise abnimmt, nämlich entsprechend dem zunehmend verzögerten Beginn der zweiten Einschaltzeit. Insoweit ist in allen fünf Steuerungszuständen die pneumatische Beaufschlagung durch das zweite Ventil 2 auch hinsichtlich der Abbremsung des Projektils 8 aktiv.

In den in Figur 4 dargestellten Fällen werden Aufschlaggeschwindigkeiten des Projektils 8 auf dem Applikator 6 von (in dieser Reihenfolge von a) zu e)) 10 m/s, 12 m/s, 14 m/s, 16 m/s und 18 m/s realisiert. Das entspricht Impulsen von 30 gm/s bis 54 gm/s bei einer Projektilmasse von 3 g. Die Öffnungszeit des Ventils 1 beträgt konstant 13,0 ms. Konstant bleibt auch der Schließzeitpunkt des zweiten Ventils bei 18 ms.

Genau genommen zeigen die Figuren 4a) bis e) die elektrischen Steuerzeiten der beiden Ventile 1 und 2, also die Ausgangssignale der Steuerung 54. Die Ventile 1 und 2 sind federunterstützte Magnetventile, die rein magnetisch öffnen und durch die Kraft der dabei gespannten Feder schließen, wenn der Magnet nicht mehr beaufschlagt wird. Die Bewegungen des Ventilkörpers sind dementsprechend gegenüber den dargestellten Steuersignalen etwas verzögert, und zwar um schätzungsweise 4 ms beim Öffnen und 2 ms beim Schließen. Die Überlappzeiten sind also tatsächlich ungefähr 2 ms kürzer als dargestellt.

Bei einem sogenannten Pilotventil mit pneumatischer Unterstützung beim Öffnen wäre die Situation qualitativ vergleichbar.

In Figur 4a) (natürlich bei einem Start der Projektilbewegung am linken Ende der Bewegungsstrecke in Figur 2 bei 0 ms) erfolgt die Kollision mit dem Applikator ungefähr am Ende der zweiten Öffnungszeit, also bei ungefähr 19 ms, wobei sich dieser Kollisionszeitpunkt in den folgenden Figuren immer weiter nach links verlagert und in Figur 4e) z. B. ungefähr bei 16 ms bis 17 ms liegt, also eher innerhalb der zweiten Öffnungszeit. Die (in einem Versuchsaufbau optisch) gemessenen Projektilgeschwindigkeiten betragen ja zwischen 10 m/s in Figur 4a) und 18 m/s in Figur 4e) und stehen damit in einem Verhältnis von 1:1,8.

Man kann sich dabei vereinfacht vorstellen, dass das Projektil vor der zweiten Einschaltzeit linear über die Zeit beschleunigt wird und danach mit etwa der erreichten Geschwindigkeit weiter bewegt wird (unter Vernachlässigung von pneumatischen Strömungseffekten und Projektilreibung); tatsächlich wird die Projektilgeschwindigkeit mit der Zeit wohl etwas weniger als linear zunehmen und in einem angenähert kräftefreien Zustand während der Überlappzeit reibungsbedingt leicht abnehmen. Außerdem gibt es in den einzelnen dargestellten Fällen jeweils eine Schlussphase, in der das Projektil 8 durch die pneumatische Beaufschlagung durch das zweite Ventil abgebremst wird. Diese Abbremsung ist bei den einzelnen Darstellungen nur insoweit leicht verschieden (nämlich in den Figuren 4d) und 4e) im Vergleich zu den vorherigen) als der Kollisionszeitpunkt leicht in die zweite Ventilöffnungszeit hineinwandert.

In den Einzeldarstellungen liegt die Überlappzeit immer vor der Kollision, ist aber unterschiedlich lang und beeinflusst insoweit die Kollisionsgeschwindigkeit. Auch die zweite Öffnungszeit liegt ganz oder zum allergrößten Teil vor der Kollision. Das stört nicht weiter, weil die Kollision selbst im Sinn des Stoßes zwischen einem typischerweise masseärmeren Projektil und einem massestärkeren Applikator im Sinn der Impulserhaltung zurückgestoßen wird. Der Rest der zweiten Einschaltzeit nach Ende der ersten Einschaltzeit bremst das Projektil genau genommen unterschiedlich stark ab, weil das sich bewegende Projektil in den verschiedenen Darstellungen in Folge der Variabilität der Überlappzeit an unterschiedlichen Orten entlang der Bewegungsstrecke und mit unterschiedlichen Geschwindigkeiten (zu Beginn der Abbremsung) erfasst wird.

Natürlich könnte man die Steuerzeiten insoweit anpassen, dass die Überlappzeit ungefähr jeweils zum Kollisionszeitpunkt endet. Insbesondere könnte das mit einer zeitlichen Bestimmung des Kollisionszeitpunkts durch die anhand Figur 2 bereits veranschaulichte Möglichkeit einer Messspule 31 in der Nähe des Applikators 6 geschehen. Wenn der Kollisionszeitpunkt relativ genau am Ende der Überlappzeit liegen soll (oder an einem anderen festgelegten Punkt), würde das Steuerzeitenschema etwas komplizierter, weil die erste Einschaltzeit unterschiedlich früh beendet werden müsste (von Figur 4a) zu Figur 4e) immer früher). Es könnte allerdings die Geschwindigkeit der Projektilbewegung, insbesondere der Rückbewegung erhöht werden. Dabei könnte es bei den höheren Projektilgeschwindigkeiten auch noch interessant sein, das Ende der zweiten Einschaltzeit unterschiedlich und bei zunehmender Projektilgeschwindigkeit früher vorzusehen, um einen noch höheren Wiederholfrequenzbereich zu erzielen.

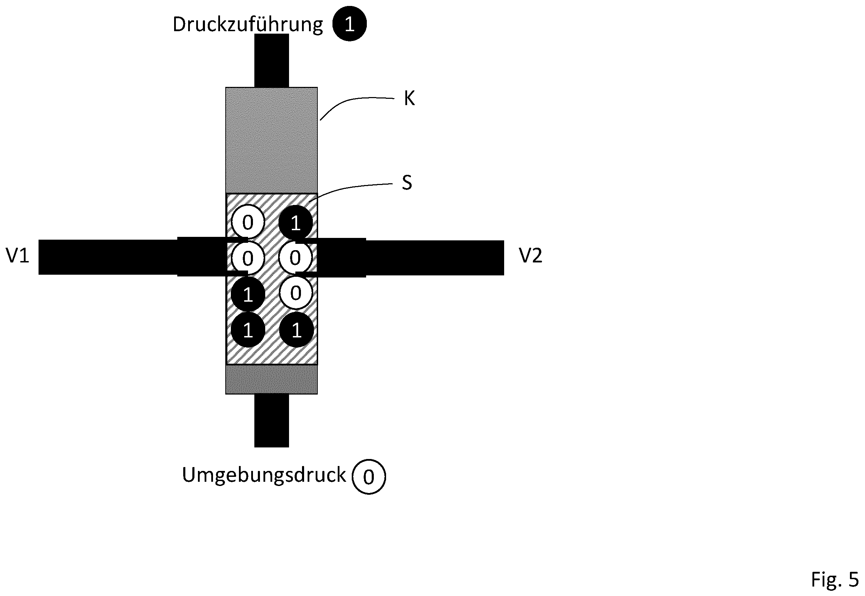

Natürlich kann man bei einem anderen Ausführungsbeispiel mit einem "Kombinationsventil" ganz ähnliche Verhältnisse erzeugen wie in Figur 4 in den Diagrammen a) bis e) dargestellt, wobei dann aber die Überlappzeit einen anderen Schaltzustand des Ventils bedeuten würde. Ein solches Kombinationsventil ist in Figur 5 schematisch dargestellt. Dabei bezeichnet der Buchstabe K das Kombinationsventil, das dementsprechend die beiden Ventile 1 und 2 aus den Figuren 1 und 2 ersetzt. Rechts und links sind zwei Leitungen V1 und V2 dargestellt, von denen V1 einen Anschluss an die linke Seite (gemäß Figur 2) des Projektilführungsrohrs 7, z. B. über das Kanalstück 22 (analog zu dem ersten Ventil 1) bedeutet. Dementsprechend bedeutet die rechte Leitung V2 einen Anschluss an die rechte Seite des Projektilführungsrohrs 7 (analog zu dem zweiten Ventil 2), also z. B. über das Kanalstück 25.

Die obere Leitung ist in Figur 5 mit dem Stichwort "Druckzuführung" und dem Symbol "1" (nicht zu verwechseln mit dem Bezugszeichen 1) für das erste Ventil bezeichnet; analog ist der untere Leitungsanschluss mit dem Stichwort "Umgebungsdruck" und dem figureninternen Symbol "0" bezeichnet, bedeutet also eine Belüftungsöffnung.

In dem Kombinationsventil K gibt es einen symbolisch dargestellten Schieber S, der in der vertikalen Richtung (bezogen auf Figur 5) zwischen vier verschiedenen Schaltpositionen verschoben werden kann. In der obersten ist, wie die Figur 5 verdeutlicht, der Anschluss V1 belüftet und der Anschluss V2 mit dem pneumatischen Versorgungsdruck beaufschlagt, in der dritten von oben umgekehrt und in der gerade aktiv geschalteten zweiten Position von oben sind beide Anschlüsse V1 und V2 belüftet. Schließlich zeigt die unterste Position eine gleichzeitige Druckbeaufschlagung beider Anschlüsse V1 und V2.

Man könnte sich also ein in dieser oder ähnlicher Weise aufgebautes Kombinationsventil K anstelle der beiden einzelnen Ventile 1 und 2 aus dem Ausführungsbeispiel in den Figuren 1 und 2 vorstellen, wobei die übrigen Erläuterungen und insbesondere die Figuren 3 und 4 sinngemäß auch dafür gelten.

Wegen der Steuerungsmöglichkeit der Aufschlaggeschwindigkeit des Projektils 8 allein über den Schaltbetrieb der beiden Ventile 1 und 2 läuft der Pneumatikkompressor 53 (Figur 3) bei einer vorgegebenen festen Betriebsfrequenz, in der er ein Wirkungsgradmaximum hat. Außerdem kann der Pneumatikkompressor bei einer vorgegebenen Betriebsfrequenz besonders wirksam schwingungs- und geräuschgedämmt werden.

Grundsätzlich kann die Steuereinrichtung 54 die Aufschlaggeschwindigkeit und auch den Zeitabstand zwischen den Kollisionen zwischen dem Projektil 8 und dem Applikator 6 von einem zu nächsten Einzelvorgang verändern. Sie kann also deutlich schneller und variabler die Stoßphysik beeinflussen und ist insbesondere nicht an periodische Vorgänge gebunden.

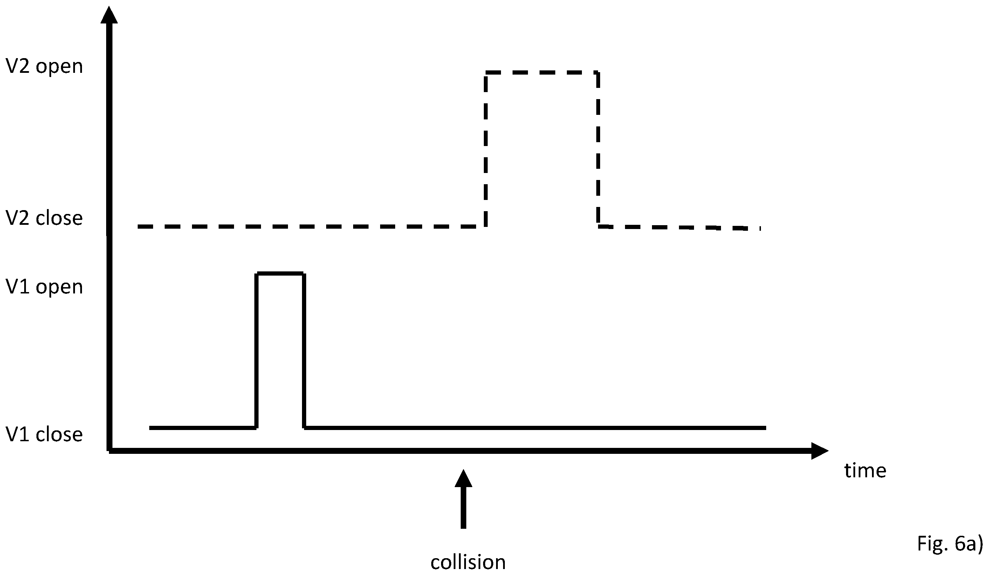

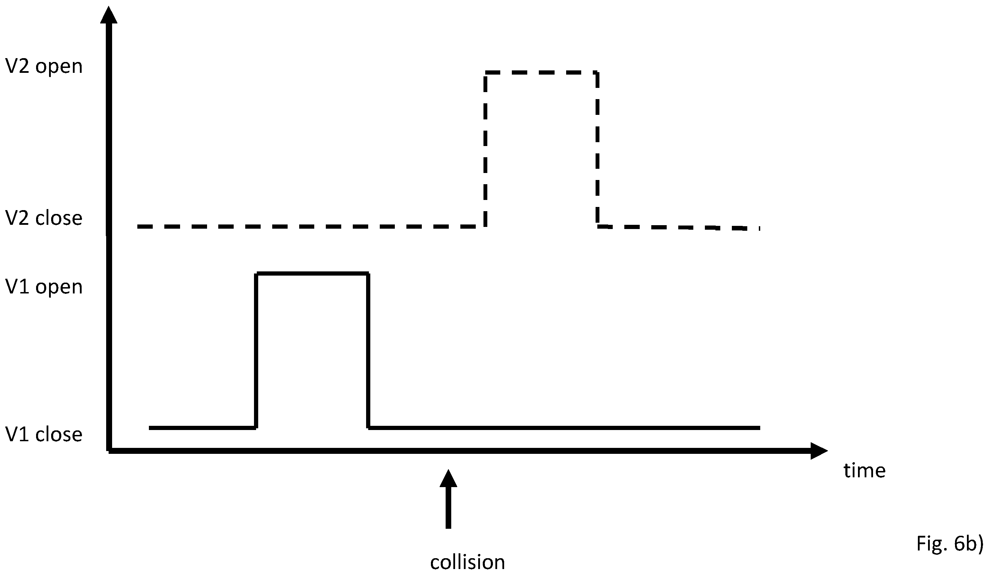

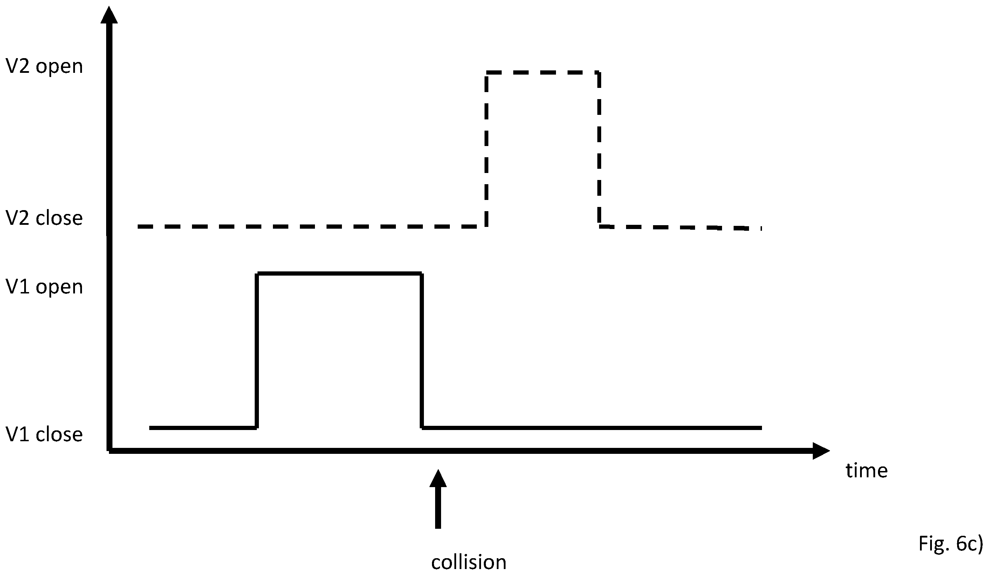

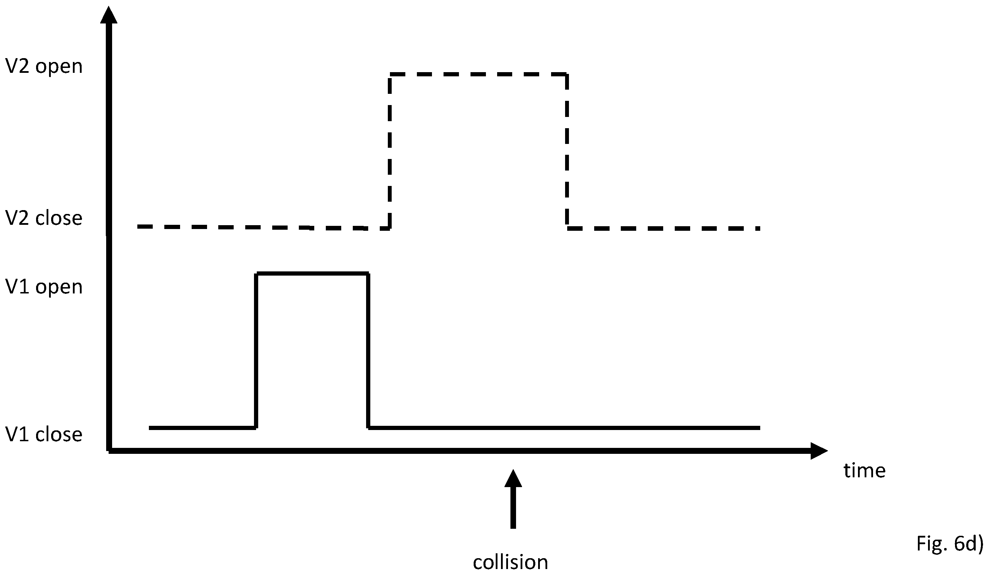

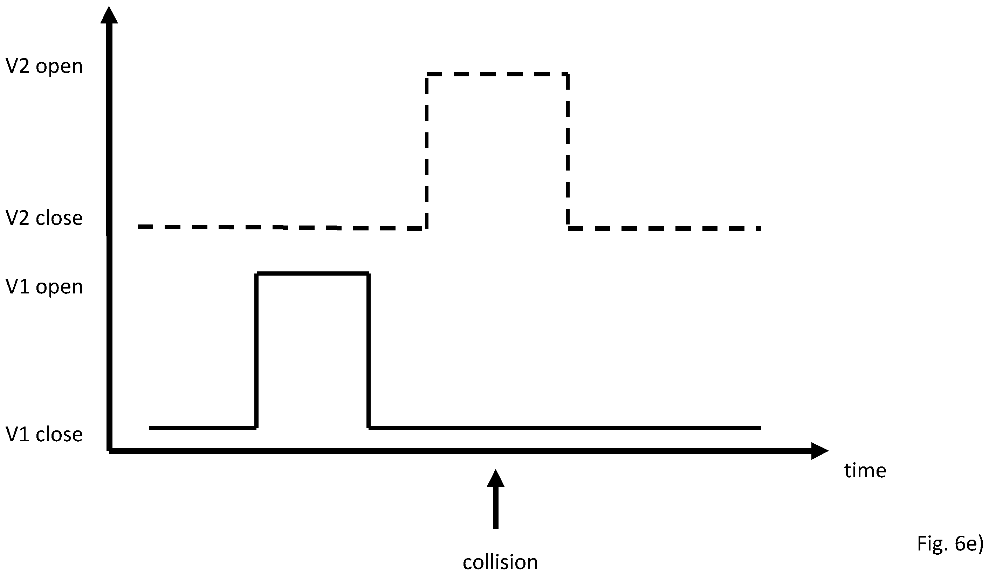

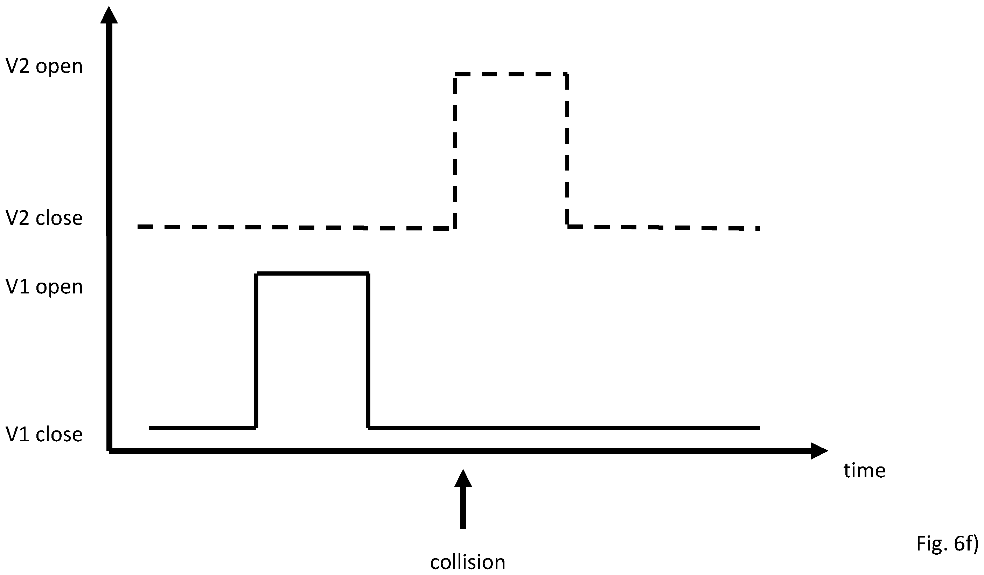

Figur 6 zeigt in den Einzeldarstellungen a) bis c) ähnliche schematische Zeitverlaufsdiagramme wie Figur 4, jedoch mit einer Abstandszeit zwischen der in durchgezogener Linie unten repräsentierten Ansteuerung des Ventils 1 aus den Figuren 1 und 2 und der in gestrichelter Linie oben dargestellten Ansteuerung des Ventils 2. In Figur 6a) gibt es einen relativ kurzen Einschaltpuls für das Ventil 1, womit das Projektil beschleunigt wird und dann nach dem Ende dieser ersten Einschaltzeit für einen wesentlichen Teil der Bewegungsstrecke ohne weitere pneumatische Beaufschlagung "weiterfliegt". Im Unterschied zu den in Figur 4 dargestellten Überlappzeiten sind jedoch in dieser Bewegungsphase beide Seiten des Rohrinneren belüftet (und nicht druckbeaufschlagt).

Nach einer bestimmten Zeit kommt es zu einer in Figur 6a) symbolisch eingezeichneten Kollision mit dem Applikator und relativ kurz danach (zusätzlich zu der bereits angedeuteten Rückbewegung des Projektils allein aufgrund dieser Kollision) zur einem rückführenden pneumatischen Puls infolge der zweiten Einschaltzeit gemäß der gestrichelten Linie in Figur 6a). Damit wird das Projektil wieder in die Ausgangslage zurückbewegt und ist für einen neuen Zyklus verfügbar.

In den Einzeldarstellungen b) und c) gilt die Erläuterung gerade eben im Prinzip genauso, wobei die erste Einschaltzeit schrittweise verlängert und damit die Abstandszeit zwischen der ersten und der zweiten Einschaltzeit schrittweise verkürzt wird. Konsequenterweise rückt der Kollisionszeitpunkt etwas nach links, was symbolisch dargestellt ist. Dementsprechend trifft das Projektil mit immer größerer Geschwindigkeit auf dem Applikator auf.

In allen drei Diagrammen a) bis c) liegt die Einschaltzeit des zweiten Ventils nach der Kollision und ist in diesen drei Diagrammen (für sich betrachtet) nicht variabel. In den ersten beiden Steuerungszuständen in Figur 6a) und b) liegt der größere Teil der Abstandszeit vor der Kollision, im dritten Fall c) danach.

In den Figuren 6d) bis f) wird im Unterschied zu den Figuren 6a) bis c) die Länge der ersten Einschaltzeit unverändert gelassen (und entspricht Figur 6b)). Im Unterschied zu den ersten drei Darstellungen ist aber die zweite Einschaltzeit variabel und liegt ein Teil der zweiten Einschaltzeit vor der Kollision, und zwar im Fall d) der überwiegende Teil, im Fall e) ungefähr die Hälfte und im Fall f) nur noch ein ganz kleiner Anteil. Man kann sich gewissermaßen als Fortsetzung die Darstellung Figur 6b) denken, in der dann die zweite Einschaltzeit vollständig nach der Kollision liegt, worauf es jetzt aber nicht besonders ankommt.

Diese Darstellungen veranschaulichen eine weitere Möglichkeit der Steuerung der Geschwindigkeit des Projektils bei der Kollision. In Figur 6d) wird nämlich das Projektil ähnlich wie in Figur 6b) über die erste Einschaltzeit hinweg pneumatisch beschleunigt, fliegt dann aber im Unterschied zum Fall b) nur relativ kurz kräftefrei, um sodann von einem entgegenstehenden pneumatischen Druck infolge des Beginns der zweiten Einschaltzeit (gestrichelt oben) verzögert zu werden. Da im Fall d) die Verzögerungszeit ungefähr der Beschleunigungszeit entspricht und von der gleichen Druckhöhe ausgegangen werden kann, schlägt das Projektil mit einer minimalen Geschwindigkeit auf dem Applikator auf und wird danach von dem Rest der zweiten Einschaltzeit wieder zurückbewegt.

In den Fällen e) und f) ist die Abstandszeit zwischen den beiden Einschaltzeiten größer und damit der Anteil der zweiten Einschaltzeit vor der Kollision schrittweise kleiner, was trotz unveränderter erster Einschaltzeit zu einer zunehmenden Projektilgeschwindigkeit bei der Kollision führt.

Insoweit muss man sich eine Steuerung (gemäß Figur 3) vorstellen, die Steuerungszustände gemäß den Teildarstellungen in Figur 4 und weitere Steuerungszustände gemäß den gerade erläuterten Teildarstellungen in Figur 6 einstellen kann. In beiden Fällen kann die Projektilgeschwindigkeit bei der Kollision durch Ventilschaltzeitpunkte bei gleichbleibendem Druck beeinflusst werden, wobei, wie schon erläutert, mit Ausnahme allein der Einzeldarstellungen in Figur 6a) bis c) ansonsten die zweite Einschaltzeit zwischen den verschiedenen Steuerungszuständen variabel ist.

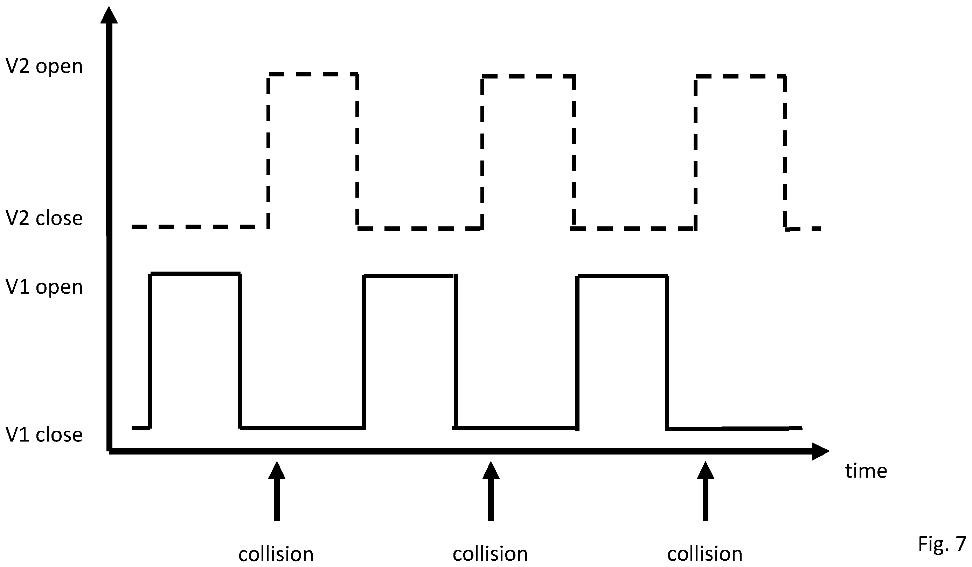

Figur 7 zeigt ungefähr eine Folge von drei Vorgängen entsprechend Figur 6f). Dabei wird durch die gestrichelt eingezeichneten zweiten Einschaltzeiten das Projektil jeweils wieder in die Ausgangslage zurückgebracht, um dann von der zeitlich folgenden ersten Einschaltzeit wieder Richtung Applikator beschleunigt zu werden. Diese Figur soll lediglich die mögliche Periodizität von Steuerungszuständen veranschaulichen, was in analoger Weise natürlich auch für die anderen Teildarstellungen in den Figuren 4 und 6 gilt. Außerdem kann man sich vorstellen, dass die aufeinanderfolgenden Prozesse Abweichungen voneinander haben können, sodass also der Stoßprozesses von einem zum nächsten Wiederholungsvorgang schnell und frei verändert werden kann.

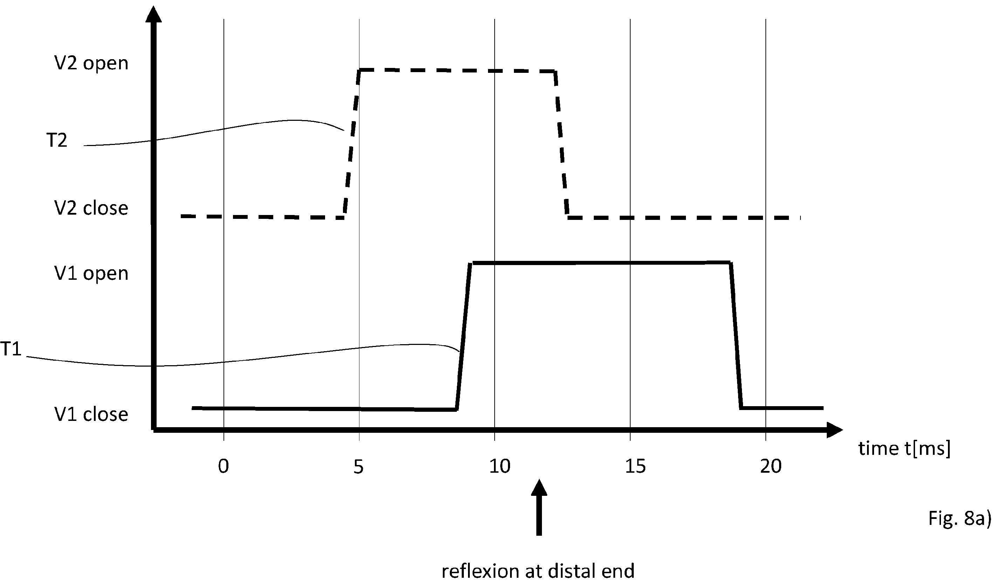

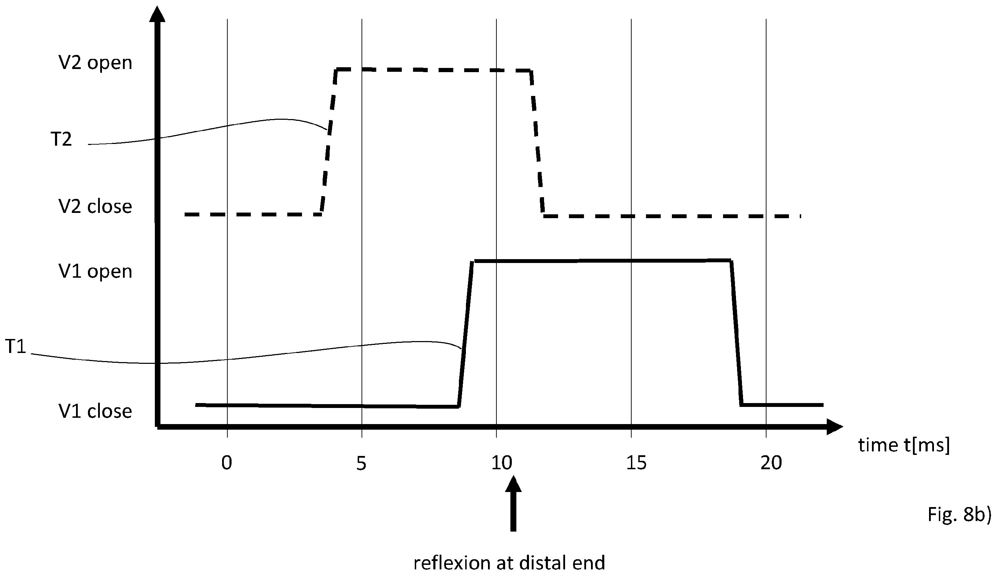

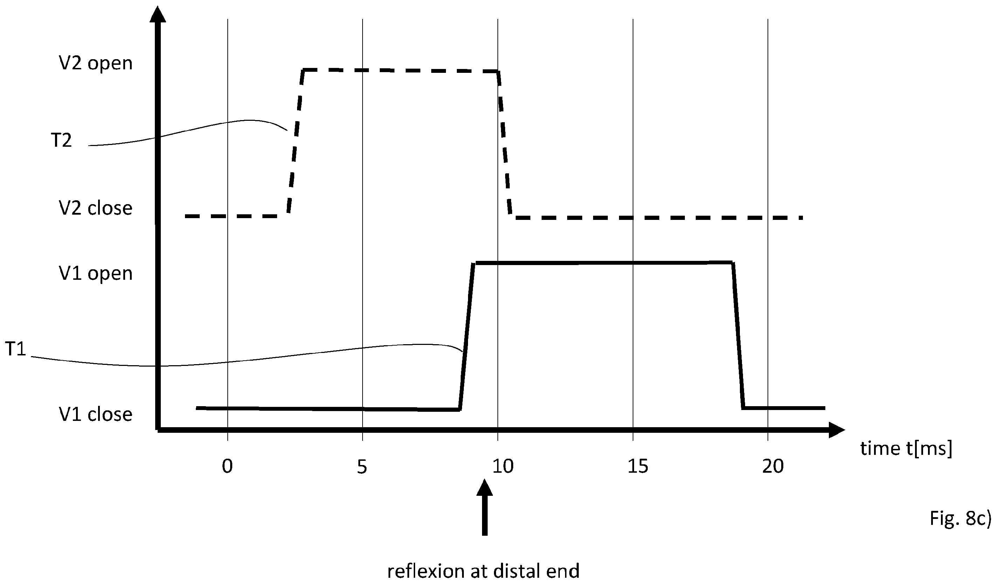

Figur 8 zeigt eine Folge von drei einzelnen schematischen Zeitverlaufsdiagrammen 8a) bis c) in denen jeweils mit der mit T1 bezeichneten Kurve der Öffnungs- und Schließvorgang des ersten Ventils 1 und mit der mit T2 bezeichneten Kurve analog der Öffnungs- und Schließvorgangs des zweiten Ventils T2 dargestellt ist. Der erhöhte Kurventeil entspricht also jeweils der ersten bzw. zweiten Einschaltzeit. Im Vergleich zu den Zeitverlaufsdiagrammen aus Figur 4 wird hier das zweite Ventil entsprechend der Kurve T2 zeitlich vor dem ersten Ventil entsprechend der Kurve T1 geöffnet. Durch Variation des Überlapps zwischen den beiden Einschaltzeiten erfolgt die Reflexion am distalen Ende der Bewegungsstrecke früher oder später, wie an der horizontalen Achse in den drei Figuren eingezeichnet. Bei diesem Beispiel sind beide Einschaltzeiten jeweils für sich (im Vergleich der drei Einzeldarstellungen miteinander) jeweils gleich lang. Allerdings verschiebt sich die zweite Einschaltzeit von Figur 8a) zu Figur 8b) und dann zu Figur 8c) relativ zur ersten Einschaltzeit weiter nach vorne, sodass die Überlappzeit abnimmt. Weil der für die Rückbeschleunigung wirksame Anteil der zweiten Ventilöffnungszeit (vor der Überlappzeit) in Figur 8c) größer als in Figur 8b) und dort wiederum größer ist als in Figur 8a), ist die bei der Reflexion am distalen Ende vorliegende Projektilgeschwindigkeit dementsprechend größer. Damit bewegt sich das Projektil nach der Reflexion am distalen Ende auch mit einer entsprechend größeren Geschwindigkeit wieder in Richtung Applikator. Darüber hinaus ist auch der für die entsprechende zusätzliche Beschleunigung wirksame Anteil der ersten Ventilöffnungszeit (nach der Überlappzeit) größer, wie der Vergleich der Figuren 8a) bis c) zeigt, sodass die Kollisionsgeschwindigkeit bei der Kollision mit dem Applikator von Figur 8a) zu Figur 8b) und schließlich zu Figur 8c) aus zwei Gründen zunimmt.

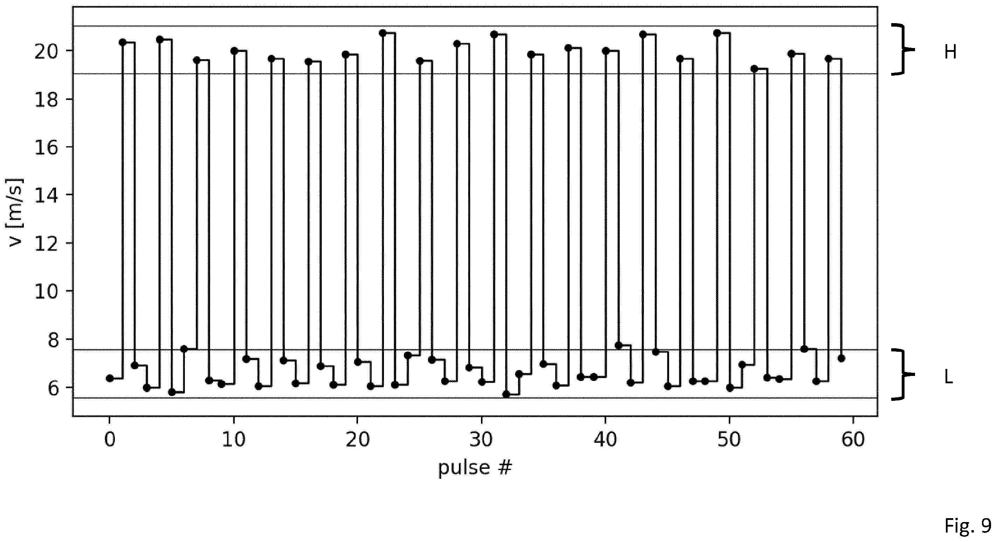

Figur 9 zeigt eine wiederkehrende Folge von Pulsen mit zwei unterschiedlichen Projektilgeschwindigkeitsbereichen (beim Aufprall), die in der Figur 9 durch die Bezugszeichen H und L gekennzeichnet sind. Durch Variation von Überlappzeit und Abstandszeit kann hier exemplarisch die Leistungsfähigkeit der Steuerung gezeigt werden. Auf jeweils einen Puls mit einer Projektilkollisionsgeschwindigkeit etwa im Bereich H kommen zwei Pulse mit einer Projektilkollisionsgeschwindigkeit etwa im Bereich L. Figur 9 demonstriert insbesondere, dass die Kollisionsbedingungen von einer Kollision zur nächsten wesentlich geändert werden können, hier mit ungefähr einem Faktor 3 in der Kollisionsgeschwindigkeit. Die Schwankungen innerhalb der Bereiche H und L sind dabei unbeabsichtigte und toleranzbedingte Streuungen (es handelt sich um reale Messwerte).

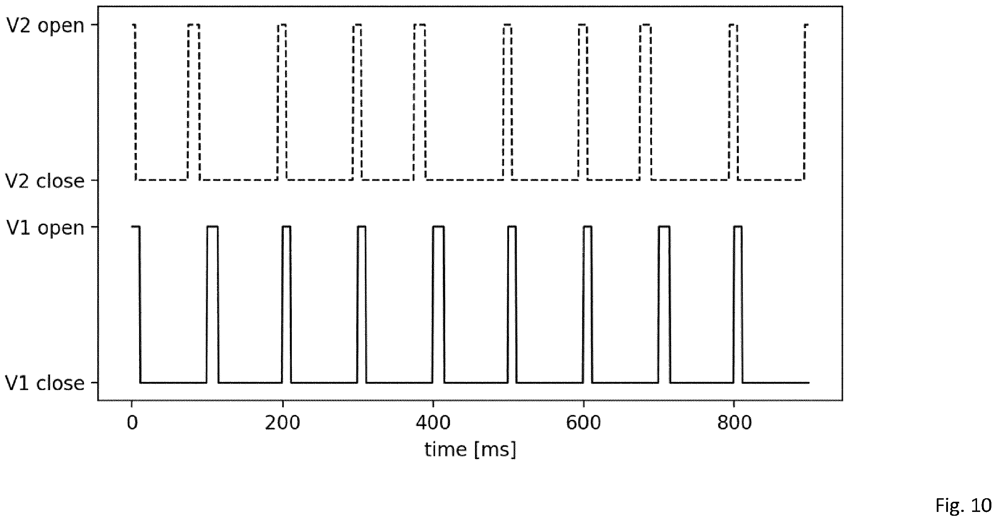

Figur 10 zeigt exemplarisch die Steuersequenz für die Ventile V1 und V2 in ihrer zeitlichen Abfolge, um die Projektilgeschwindigkeitsfolgen zu erzielen, welche in Figur 9 zu sehen sind. Es sind unterschiedliche Überlappungen und Abstände von Pulsen relativ zueinander zu sehen.

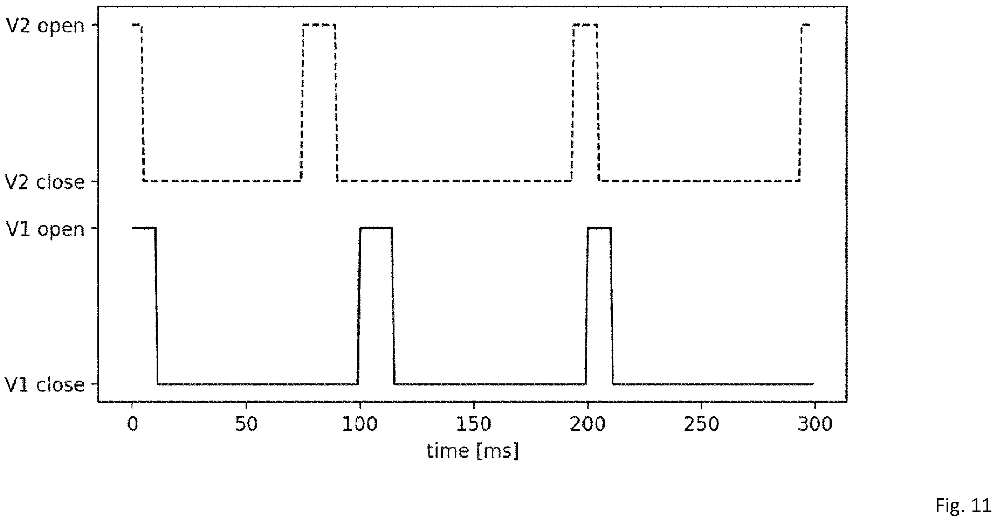

Figur 11 löst die Abfolge der ersten Pulse aus Figur 10 zeitlich genauer auf, sodass hier eine sich wiederholende Sequenz einzeln gezeigt ist. Hier ist deutlicher zu sehen, dass die Ventilöffnungszeiten zwischen V1 und V2 relativ ihren Abstand und Überlapp ändern und dass sich die zweite Einschaltzeit ändert.

Die obigen Erläuterungen anhand der Figuren 4 sowie 6 bis 11 beziehen sich auf das in den Figuren 1 bis 3 dargestellte Gerät. Sie lassen sich anhand einfacher Abschätzungen zur Projektilbewegung auch auf andere Geräte und Dimensionen übertragen. Insbesondere sind die Umkehrpunkte der Projektilbewegung bspw. über die erwähnte Messspule, evtl. eine analoge Messspule am distalen Ende der Bewegungsstrecke oder über die Erfassung der Kollisionen per Mikrophon, leicht zugänglich. Auf dieser Grundlage lassen sich anhand der obigen Schilderungen sinnvolle Abschätzungen treffen.

Alternativ kann folgendermaßen vorgegangen werden: Man gibt eine gewünschte Betriebsfrequenz und einen gewünschten Versorgungsdruck für die beiden Ventile vor und gibt z. B. auch vor, dass beide Ventile für konstante Dauer öffnen, z. B. für 40 % des Kehrwerts der vorgegebenen Frequenz. Dann kann man die Steuerung so einrichten, dass die Ventile in einem Startzeitpunkt genau gleichphasig öffnen und schließen. In diesem Zustand wird keine stabile Bewegung zustande kommen, weil das Projektil zeitgleich beidseits mit Druck beaufschlagt wird bzw. von keiner Seite mit Druck beaufschlagt wird. Auf dieser Grundlage kann man dann den Versatz zwischen Öffnungszeitpunkten in beide Richtungen schrittweise verändern, also schrittweise das zweite Ventil etwas früher oder etwas später als das erste Ventil öffnen (und schließen). Ab einem gewissen Zeitversatz, also sozusagen ab einer gewissen Phasenverschiebung, wird es zu einem stabilen Schwingungszustand des Projektils kommen, was man z. B. mit der erwähnten Mikrophonermittlung der Kollisionen an den beiden Enden der Bewegungsstrecke feststellen kann. Außerdem kann man dann die Intensität der Kollision mit dem Applikator ermitteln und die beschriebene Phasenverschiebung gewissermaßen als Stellparameter für die Intensität betrachten. In dieser Form lässt sich eine Eichkurve ermitteln.

Außerdem kann man bei einem bestimmten in dieser Form ermittelten Schwingungszustand den Phasenversatz konstant halten und die zweite Ventilöffnungsdauer (und möglicherweise oder stattdessen auch die erste) schrittweise verändern.

Im Einzelfall könnte es dazu kommen, dass für die gewünschte Frequenz kein ausreichender Druck vorgegeben wurde, also auch bei "gegenphasiger" Ansteuerung der beiden Ventile kein Schwingungszustand mit Kollisionen an den Enden der Bewegungsstrecke entsteht. Dann muss dementsprechend entweder etwas der Druck erhöht oder die Frequenz verringert werden.

Analog kann man sich natürlich auch in anderer Form an geeignete Betriebszustände empirisch herantasten. Schließlich lässt sich natürlich das Bewegungsverhalten des Projektils zumindest näherungsweise rechnerisch simulieren und es können dann empirische Versuche ausgehend von den Resultaten solcher Simulationen unternommen werden.

## Patentansprüche

1. Gerät zur Behandlung des menschlichen oder tierischen Körpers mit mechanischen Druckwellen, welches Gerät aufweist:
- ein Projektil, das in dem Gerät entlang einer Bewegungsstrecke geführt ist,
- einen Applikator (6) an einem Ende der Bewegungsstrecke,
- eine pneumatische Einrichtung zur Beaufschlagung des Projektils (8) mit pneumatischem Druck zwecks einer Bewegung entlang der Bewegungsstrecke,
wobei das Projektil (8) zu einem Aufschlagen auf den Applikator (6) zur Erzeugung der mechanischen Druckwellen ausgelegt ist,
welche pneumatische Einrichtung eine Doppelventileinrichtung (1, 2) zur Beaufschlagung des Projektils (8) mit pneumatischem Druck in Richtung zu dem Applikator (6) hin während einer ersten Einschaltzeit und zur Beaufschlagung des Projektils (8) mit pneumatischem Druck in der Rückrichtung während einer zweiten Einschaltzeit sowie eine Steuereinrichtung (54) zur Ansteuerung der Doppelventileinrichtung (1, 2) aufweist,
**dadurch gekennzeichnet, dass** das Gerät dazu ausgelegt ist, die zweite Einschaltzeit zu variieren.

2. Gerät nach Anspruch 1, bei dem die Doppelventileinrichtung (1, 2) ein erstes Ventil (1) zur Beaufschlagung des Projektils (8) mit pneumatischem Druck in Richtung zu dem Applikator (6) hin und ein zweites Ventil (2) zur Beaufschlagung des Projektils (8) mit pneumatischem Druck in der Rückrichtung aufweist, die vorzugsweise unabhängig voneinander von der Steuereinrichtung (54) ansteuerbar sind.

3. Gerät nach Anspruch 2, bei dem mindestens eines der beiden Ventile (1, 2) ein Zweiwegeventil ist, das ein pneumatisches Volumen zwischen sich und dem Projektil (8) in einer ersten Schaltstellung während der jeweiligen Einschaltzeit zur Beaufschlagung des Projektils (8) mit pneumatischem Druck beaufschlagt und das in einer zweiten Schaltstellung dieses pneumatische Volumen belüftet.

4. Gerät nach einem der vorstehenden Ansprüche, dazu ausgelegt, die zweite Einschaltzeit zur Steuerung einer Aufschlaggeschwindigkeit des Projektils (8) bei dem Aufschlagen zu variieren.

5. Gerät nach Anspruch 4, dazu ausgelegt, die zweite Einschaltzeit nach einem Bewegungsstart des Projektils (8) in der Hinrichtung zu dem Applikator (6) und vor dem Aufschlagen zu beginnen und nach dem Aufschlagen zu beenden und die Aufschlaggeschwindigkeit durch Variation des Anfangszeitpunkts der zweiten Einschaltzeit zu steuern.

6. Gerät nach Anspruch 4 oder 5, dazu ausgelegt, die zweite Einschaltzeit für eine Rückbewegung des Projektils (8) zu nutzen und über den Neubeginn einer Bewegung des Projektils (8) in der Hinrichtung zu dem Applikator (6) hinaus andauern zu lassen.

7. Gerät nach einem der vorstehenden Ansprüche, bei dem die zweite Einschaltzeit während einer Rückbewegung des Projektils (8) variiert wird.

8. Gerät nach einem der vorstehenden Ansprüche, bei dem die Steuereinrichtung (54) dazu ausgelegt ist, die Länge der zweiten Einschaltzeit beim Steuern zu variieren.

9. Gerät nach Anspruch 8, bei dem die Steuereinrichtung (54) dazu ausgelegt ist, das Ende der zweiten Einschaltzeit beim Steuern konstant zu halten.

10. Gerät nach einem der vorstehenden Ansprüche, dazu ausgelegt, die erste und die zweite Einschaltzeit in einer Überlappzeit überlappen zu lassen.

11. Gerät nach einem der vorstehenden Ansprüche, bei dem die pneumatische Einrichtung einen Pneumatikkompressor (53) aufweist, wobei das Gerät dazu ausgelegt ist, den Kompressor (53) im eingeschalteten Zustand bei verschiedenen Steuerungszuständen mit unterschiedlichen Aufschlaggeschwindigkeiten des Projektils (8) bei gleicher Drehzahl laufen zu lassen, vorzugsweise grundsätzlich im eingeschalteten Zustand bei immer der gleichen Drehzahl laufen zu lassen.

12. Gerät nach einem der vorstehenden Ansprüche, dazu ausgelegt, dass bei einer Bewegung des Projektils (8) von einem von dem Applikator (6) distalen Ort der Bewegungsstrecke zu dem Applikator (6) und zurück der auf die Hinbewegung entfallende Zeitraum der Überlappzeit größer als der auf die Rückbewegung entfallende Zeitraum ist.

13. Gerät nach einem der vorstehenden Ansprüche, welchem das Projektil (8) mit einem Aufschlagimpuls von zwischen 2 gm/s und 300 gm/s beim Aufschlag auf den Applikator (6) bewegt werden kann.

14. Gerät nach einem der vorstehenden Ansprüche, dazu ausgelegt, in einem iterativen Betriebszustand mit unmittelbar aufeinanderfolgenden Hinbewegung des Projektils (8) zum Aufschlag auf den Applikator (6) und Rückbewegungen die Aufschlaggeschwindigkeit und/oder die Zeitdauer der kombinierten Hin- und Rückbewegung von einer zur nächsten solchen kombinierten Hin- und Rückbewegung zu verändern.

15. Gerät nach einem der vorstehenden Ansprüche mit einer Messeinrichtung (31) zur Erfassung eines Durchtritts und/oder einer Geschwindigkeit des Projektils (8) an einer Stelle der Bewegungsstrecke, welche Messeinrichtung (31) mit der Steuereinrichtung (54) gekoppelt ist.

## Claims

1. Apparatus for treatment of the human or animal body with mechanical pressure waves, the apparatus comprising:
- a projectile guided in the apparatus along a movement path,
- an applicator (6) at one end of the movement path,
- pneumatic means for application of pneumatic pressure to the projectile (8) for the purpose of movement along the movement path,
wherein the projectile (8) is adapted for striking onto the applicator (6) for generating the mechanical pressure waves,
which pneumatic means has a double valve means (1, 2) for application of pneumatic pressure to the projectile (8) in the direction towards the applicator (6) during a first activation time and for application of pneumatic pressure to the projectile (8) in the reverse direction during a second activation time and a control means (54) for controlling the double valve means (1, 2),
**characterized in that** the apparatus is adapted to vary the second activation time.

2. Apparatus according to claim 1, in which the double valve means (1, 2) has a first valve (1) for application of pneumatic pressure to the projectile (8) in the direction towards the applicator (6) and a second valve (2) for application of pneumatic pressure to the projectile (8) in the reverse direction, which valves can preferably be controlled independently of one another by the control means (54).

3. Apparatus according to claim 2, in which at least one of the two valves (1, 2) is a two-way valve which applies pneumatic pressure to a pneumatic volume between itself and the projectile (8) in a first switching position during the respective activation time for application of pneumatic pressure to the projectile (8) and which ventilates this pneumatic volume in a second switching position.

4. Apparatus according to one of the preceding claims, adapted to vary the second activation time for controlling an impact speed of the projectile (8) upon impact.

5. Apparatus according to claim 4, adapted to start the second activation time after a movement start of the projectile (8) in the forward direction towards the applicator (6) and before impact and to end it after impact and to control the impact speed by varying the start time of the second activation time.

6. Apparatus according to claim 4 or 5, adapted to use the second activation time for a return movement of the projectile (8) and to allow it to continue beyond the new start of a movement of the projectile (8) in the forward direction towards the applicator (6).

7. Apparatus according to one of the preceding claims, in which the second activation time is varied during a return movement of the projectile (8).

8. Apparatus according to one of the preceding claims, in which the control means (54) is adapted to vary the length of the second activation time during control.

9. Apparatus according to claim 8, in which the control means (54) is adapted to maintain the end of the second activation time constant during control.

10. Apparatus according to one of the preceding claims, adapted to allow the first and the second activation time to overlap in an overlap time.

11. Apparatus according to one of the preceding claims, wherein the pneumatic means comprises a pneumatic compressor (53), wherein the apparatus is adapted to allow the compressor (53) in the activated state to run at different control states with different impact speeds of the projectile (8) at the same rotational frequency, preferably in principle in the activated state to run at always the same rotational frequency.

12. Apparatus according to one of the preceding claims, adapted such that, in the case of a movement of the projectile (8) from a location of the movement path distal from the applicator (6) to the applicator (6) and back, the time period of the overlap time attributed to the forward movement is greater than the time period attributed to the return movement.

13. Apparatus according to one of the preceding claims, wherein the projectile (8) can be moved with an impact pulse of between 2 gm/s and 300 gm/s upon impact onto the applicator (6).

14. Apparatus according to one of the preceding claims, adapted to vary, in an iterative operating state with directly successive forward movements of the projectile (8) for impact onto the applicator (6) and return movements, the impact speed and/or the time duration of the combined forward and return movement from one to the next such combined forward and return movement.

15. Apparatus according to one of the preceding claims, having a measuring means (31) for detecting a passage and/or a speed of the projectile (8) at a point of the movement path, which measuring means (31) is coupled to the control means (54).

## Revendications

1. Appareil pour traiter le corps humain ou animal avec des ondes de pression mécaniques, l'appareil comprenant :
- un projectile guidé dans l'appareil le long d'une trajectoire de déplacement,
- un applicateur (6) à une extrémité de la trajectoire de déplacement,
- un dispositif pneumatique pour soumettre le projectile (8) à une pression pneumatique en vue d'un déplacement le long de la trajectoire de déplacement,
le projectile (8) étant conçu pour percuter l'applicateur (6) afin de générer les ondes de pression mécaniques,
le dispositif pneumatique comprenant un dispositif à double soupape (1, 2) pour soumettre le projectile (8) à une pression pneumatique en direction de l'applicateur (6) pendant un premier temps de mise en marche et pour soumettre le projectile (8) à une pression pneumatique dans la direction inverse pendant un deuxième temps de mise en marche ainsi qu'un dispositif de commande (54) pour commander le dispositif à double soupape (1, 2),
**caractérisé en ce que** l'appareil est conçu pour faire varier le deuxième temps de mise en marche.

2. Appareil selon la revendication 1, dans lequel le dispositif à double soupape (1, 2) comprend une première soupape (1) pour soumettre le projectile (8) à une pression pneumatique en direction de l'applicateur (6) et une deuxième soupape (2) pour soumettre le projectile (8) à une pression pneumatique dans la direction inverse, lesquelles peuvent être commandées de préférence indépendamment l'une de l'autre par le dispositif de commande (54).

3. Appareil selon la revendication 2, dans lequel au moins l'une des deux soupapes (1, 2) est une soupape à deux voies qui, dans une première position de commutation, soumet un volume pneumatique entre elle-même et le projectile (8) à une pression pneumatique pendant le temps de mise en marche respectif pour soumettre le projectile (8) à une pression pneumatique et qui, dans une deuxième position de commutation, ventile ce volume pneumatique.

4. Appareil selon l'une quelconque des revendications précédentes, conçu pour faire varier le deuxième temps de mise en marche pour commander une vitesse de percussion du projectile (8) lors de la percussion.

5. Appareil selon la revendication 4, conçu pour commencer le deuxième temps de mise en marche après un début de déplacement du projectile (8) dans la direction allant vers l'applicateur (6) et avant la percussion et pour le terminer après la percussion et pour commander la vitesse de percussion par variation du moment de début du deuxième temps de mise en marche.

6. Appareil selon la revendication 4 ou 5, conçu pour utiliser le deuxième temps de mise en marche pour un mouvement inverse du projectile (8) et pour le laisser durer au-delà du nouveau début d'un déplacement du projectile (8) dans la direction allant vers l'applicateur (6).

7. Appareil selon l'une quelconque des revendications précédentes, dans lequel le deuxième temps de mise en marche est varié pendant un mouvement inverse du projectile (8).

8. Appareil selon l'une quelconque des revendications précédentes, dans lequel le dispositif de commande (54) est conçu pour faire varier la longueur du deuxième temps de mise en marche lors de la commande.

9. Appareil selon la revendication 8, dans lequel le dispositif de commande (54) est conçu pour maintenir constante la fin du deuxième temps de mise en marche lors de la commande.

10. Appareil selon l'une quelconque des revendications précédentes, conçu pour laisser les premier et deuxième temps de mise en marche se chevaucher dans un temps de chevauchement.

11. Appareil selon l'une quelconque des revendications précédentes, dans lequel le dispositif pneumatique comprend un compresseur pneumatique (53), l'appareil étant conçu pour faire fonctionner le compresseur (53) à l'état mis en marche dans différents états de commande avec différentes vitesses de percussion du projectile (8) à la même vitesse de rotation, de préférence pour le faire fonctionner en principe à l'état mis en marche toujours à la même vitesse de rotation.

12. Appareil selon l'une quelconque des revendications précédentes, conçu de telle sorte que lors d'un déplacement du projectile (8) d'un emplacement de la trajectoire de déplacement distal de l'applicateur (6) vers l'applicateur (6) et inversement, la période de temps du temps de chevauchement résultant du mouvement aller est supérieure à la période de temps résultant du mouvement inverse.

13. Appareil selon l'une quelconque des revendications précédentes, dans lequel le projectile (8) peut être déplacé avec une impulsion de percussion comprise entre 2 gm/s et 300 gm/s lors de la percussion sur l'applicateur (6).

14. Appareil selon l'une quelconque des revendications précédentes, conçu pour faire varier, dans un état de fonctionnement itératif avec des mouvements aller directement successifs du projectile (8) pour la percussion sur l'applicateur (6) et des mouvements inverses, la vitesse de percussion et/ou la durée du mouvement aller et inverse combiné d'un tel mouvement aller et inverse combiné au suivant.

15. Appareil selon l'une quelconque des revendications précédentes, comprenant un dispositif de mesure (31) pour détecter un passage et/ou une vitesse du projectile (8) à un endroit de la trajectoire de déplacement, lequel dispositif de mesure (31) est couplé au dispositif de commande (54).
